(19) **Europäisches Patentamt / European Patent Office / Office européen des brevets**

(11) **EP 2 833 131 B1**

(12) # EUROPEAN PATENT SPECIFICATION

(45) Date of publication and mention
of the grant of the patent:
**01.02.2017 Bulletin 2017/05**

(51) Int Cl.:
*G01N 27/18* (2006.01)  *G01N 33/22* (2006.01)

(21) Application number: **14177755.7**

(22) Date of filing: **21.07.2014**

(54) **Calorific value measurement system and method of measuring a calorific value**

Brennwertmesssystem und Verfahren zur Messung eines Brennwerts

Système et procédé de mesure d'une valeur calorifique

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.08.2013 JP 2013160735**

(43) Date of publication of application:
**04.02.2015 Bulletin 2015/06**

(73) Proprietor: **Azbil Corporation**
**Chiyoda-ku**
**Tokyo 100-6419 (JP)**

(72) Inventor: **Ooishi, Yasuharu**
**Tokyo, Tokyo 100-6419 (JP)**

(74) Representative: **Lavoix**
**Bayerstrasse 83**
**80335 München (DE)**

(56) References cited:
**EP-A1- 1 947 450    EP-A2- 1 063 525**
**EP-A2- 2 369 338**

EP 2 833 131 B1

**Description**

Background

The present invention relates to a calorific value measurement system and a method of measuring a calorific value capable of measuring a calorific value of mixed gas.

In the past, as this kind of calorific value measurement system, there is known a system configured to calculate a calorific value of measurement target mixed gas based on a calorific value calculation formula where heat loss coefficients or thermal conductivities at a plurality of different temperatures are independent variables and a calorific value is a dependent variable (for example, see International Publication No. 2010/038285).

EP 2 369 338 A2 describes a calorific value calculation formula creation system which can measure the calorific value of a gas, a method of creating a calorific value calculation formula, a calorific value measurement system, and a method of measuring a calorific value.

Summary

According to International Publication No. 2010/038285, a calorific value measurement system is configured to introduce measurement target mixed gas in a chamber, and to measure heat loss coefficients or thermal conductivities of the mixed gas at a plurality of different temperatures. Gas (e.g., air, etc.) other than the mixed gas may remain in the chamber, or air may enter the chamber from a clearance or the like of pipes.

In this case, heat loss coefficients or thermal conductivities of gas other than the mixed gas in the chamber are measured at a plurality of different temperatures, and the measured heat loss coefficients or thermal conductivities, i.e., independent variables, are substituted in a calorific value calculation formula. However, the calorific value of gas other than the measurement target mixed gas may not be correctly calculated based on the calorific value calculation formula.

In view of the above-mentioned circumstances, it is an object of the present invention to provide a calorific value measurement system and a method of measuring a calorific value capable of distinguishing between measurement target mixed gas and gas other than the mixed gas.

According to the present invention, there is provided a calorific value measurement system configured to measure a calorific value of measurement target mixed gas, the system including: a heater element coming in contact with gas, the heater element being configured to generate heat at a plurality of different temperatures; a measurement module configured to measure a value of an electric signal from the heater element; a determination module configured to compare a determination value with a predetermined threshold, the determination value being based on a measured value of an electric signal from the heater element, the heater element generating heat at a first temperature out of the plurality of different temperatures, and to determine if the gas is measurement target or not based on a comparison result; and a calorific value calculation module configured to calculate a calorific value of the gas based on a measured value of an electric signal from the heater element if it is determined that the gas is a measurement target. The predetermined threshold is set based on a value of an electric signal from the heater element, the heater element generating heat at the first temperature, when the heater element is in contact with a sample mixed gas, and also based on a value of an electric signal from the heater element when the heater element is in contact with the air.

Further, according to the present invention, there is provided a method of measuring a calorific value, the method measuring a calorific value of measurement target mixed gas, the method including: measuring a value of an electric signal from a heater element coming in contact with gas, the heater element being configured to generate heat at a plurality of different temperatures; comparing a determination value with a predetermined threshold, the determination value being based on a measured value of an electric signal from the heater element, the heater element generating heat at a first temperature out of the plurality of different temperatures, and determining if the gas is measurement target or not based on a comparison result; and calculating a calorific value of the gas based on a measured value of an electric signal from the heater element if it is determined that the gas is a measurement target. The predetermined threshold is set based on a value of an electric signal from the heater element, the heater element generating heat at the first temperature, when the heater element is in contact with a sample mixed gas, and also based on a value of an electric signal from the heater element when the heater element is in contact with the air.

According to the calorific value measurement system and the method of measuring a calorific value of the present invention, it is possible to distinguish between measurement target mixed gas and gas other than the mixed gas. Further advantages, features, aspects and details are evident from the dependent claims, the description and the drawings.

Brief Description of Drawings

Fig. 1 is a diagram schematically showing a configuration of the calorific value measurement system according to an embodiment;

Fig. 2 is a perspective view showing a microchip of Fig. 1;

Fig. 3 is a cross-sectional view illustrating the microchip taken along the line II-II of Fig. 2;

Fig. 4 is a circuit diagram showing an example of the circuit including a heater element of Fig. 2 and Fig. 3;

Fig. 5 is a circuit diagram showing an example of a circuit including a first temperature measurement element of Fig. 2 and Fig. 3;

Fig. 6 is a circuit diagram showing an example of the circuit including a heat-retaining element of Fig. 2 and Fig. 3;

Fig. 7 is a graph showing an example of the relationship between temperature of the heater element of Fig. 2 and Fig. 3 and heat loss coefficients of gas;

Fig. 8 is a diagram schematically showing the configuration of a calorific value calculation formula creation system including the calorific value measurement system of Fig. 1;

Fig. 9 is a graph showing an example of the relationship between the heat loss coefficient and the thermal conductivity of mixed gas;

Fig. 10 is a flowchart illustrating an example of a behavior of the calorific value measurement system of Fig. 1 when the calorific value measurement system measures a calorific value of mixed gas;

Fig. 11 is a graph showing an example of the relationship between the kinds of gas and determination values;

Fig. 12 is a graph showing an example of temporal change of a determination value and a calculated calorific value;

Fig. 13 is a graph showing another example of temporal change of a determination value and a calculated calorific value; and

Fig. 14 is a graph showing still another example of temporal change of a determination value and a calculated calorific value.

Detailed Description of Embodiments

Hereinafter, an embodiment of the present invention will be described. The same or similar components are denoted by the same or similar reference symbols in the attached drawings. Note that because the drawings are schematically illustrated, for example, specific dimensions need to be determined in the light of the following description. In addition, as a matter of course, the same components may have different dimensions or ratios in the drawings. Note that, in the following description, the upper side, the lower side, the left side, and the right side of the sheets will be referred to as "upper", "bottom", "left", and "right", respectively.

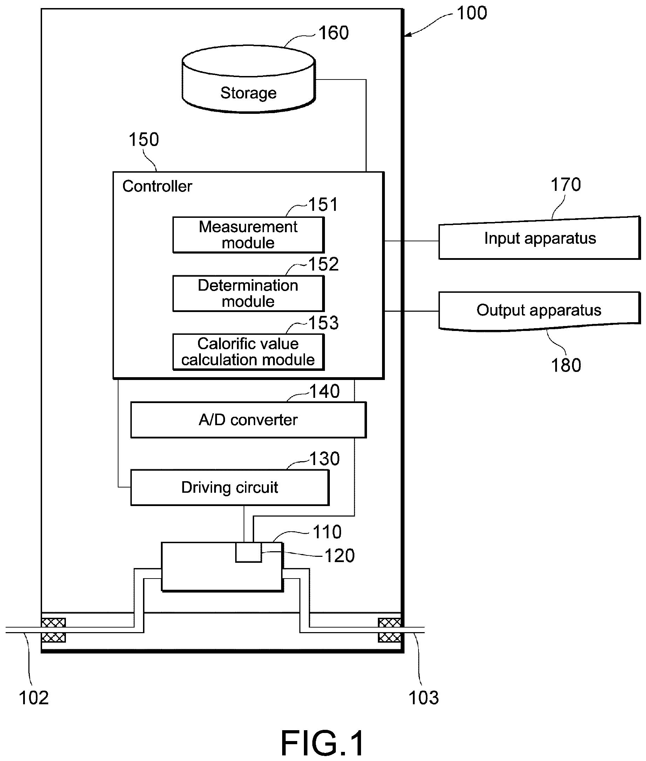

Fig. 1 to Fig. 14 show a calorific value measurement system and a method of measuring a calorific value according to an embodiment of the present invention. Fig. 1 is a diagram schematically showing the configuration of the calorific value measurement system 100 according to the embodiment. As shown in Fig. 1, the calorific value measurement system 100 is configured to measure the calorific value of mixed gas including a plurality of kinds of gas components, for example, natural gas or the like. The calorific value measurement system 100 includes the chamber 110, the microchip 120, the driving circuit 130, the A/D converter 140, the controller 150, the storage 160, the input apparatus 170, and the output apparatus 180.

The chamber 110 is a container in which gas is introduced. The flow path 102 and the flow path 103 are connected to the chamber 101. The flow path 102 transfers mixed gas to the chamber 101. The flow path 103 exhausts the sample mixed gas from the chamber 101 to the outside. Further, the microchip 120 is arranged in the chamber 110 with the insulating member 18 (described later) interposed therebetween.

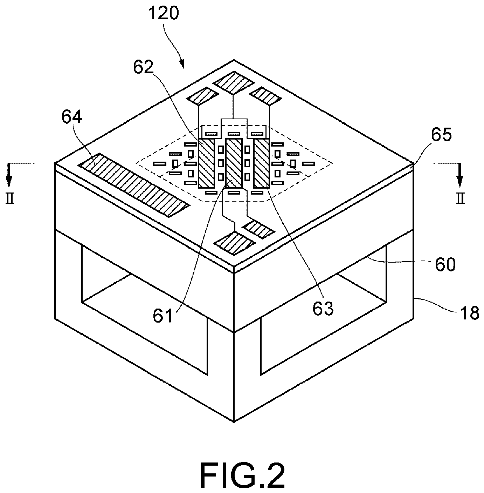

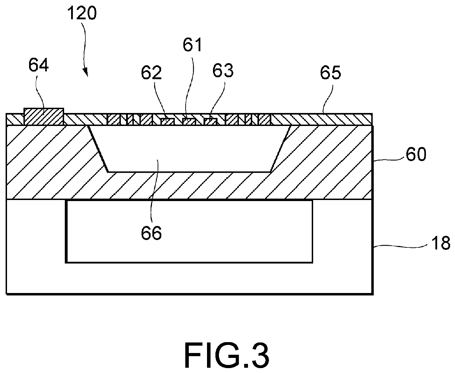

Fig. 2 is a perspective view showing the microchip 120 of Fig. 1. Fig. 3 is a cross-sectional view illustrating the microchip taken along the line II-II of Fig. 2. As shown in Fig. 2 and Fig. 3, the microchip 120 includes the substrate 60, the heater element 61, the first temperature measurement element 62, the second temperature measurement element 63, the heat-retaining element 64, and the insulating film 65.

The substrate 60 has the cavity 66. The cavity 66 is an opening at one surface (upper surface of each of Fig. 2 and Fig. 3) of the substrate 60. The thickness of the substrate 60 is, for example, 0.5 [mm]. The substrate 60 is about 1.5 [mm] square, for example. The insulating film 65 is arranged on the substrate 60 such that the insulating film 65 covers the cavity 66 of the substrate 60. The portion of the insulating film 65 covering the cavity 66 functions as a heat insulating diaphragm. The heater element 61, the first temperature measurement element 62, and the second temperature measurement element 63 are provided on the diaphragm portion of the insulating film 65. The heater element 61 is provided between the first temperature measurement element 62 and the second temperature measurement element 63. Further the heat-retaining element 64 is provided on the substrate 60.

The heater element 61 is arranged at the center of the diaphragm portion of the insulating film 65 covering the cavity 66. The heater element 61 is, for example, a resistor and is supplied with power to generate heat, to thereby heat gas in contact with the heater element 61. The first temperature measurement element 62 and the second temperature measurement element 63 are, for example, passive elements such as resistors, and output electric signals depending on the gas temperature. Hereinafter, an example in which an output signal from the first temperature measurement element 62 is used will be described, but the present invention is not limited thereto. For example, an output signal from the second temperature measurement element 63 may be used. Alternatively, an average value of an output signal from the first temperature measurement element 62 and an output signal from the second temperature measurement element 63 may be used as the output signal from the temperature measurement elements.

The heat-retaining element 64 is, for example, a resistor and is supplied with power to generate heat, to thereby keep the temperature of the substrate 60 constant. The substrate 60 may be made from silicon (Si) or the like. The insulating film 65 may be made from silicon oxide ($SiO_2$) or the like. The cavity 66 is formed by anisotropic etching or the like. In addition, the heater element 61, the first temperature measurement element 62, the second temperature measurement element 63, and the heat-retaining element 64 may be made from platinum (Pt) and may be formed by using a lithography method or the like. In addition, the heater element 61, the first temperature measurement element 62, and the second temperature measurement element 63 may be formed of the same members.

The microchip 120 is fixed on a container such as a chamber, which is to be filled up with gas, with a heat insulating member 18 provided on the bottom of the microchip 120, interposed therebetween. When the microchip 120 is fixed on, for example, the chamber with the heat insulating member 18 interposed therebetween, the temperature of the microchip 120 is less likely to be affected by variation in temperature of the inner wall and the like of the chamber. For example, the thermal conductivity of the heat insulating member 18 made of glass or the like is equal to or less than 1.0 [W/(m·K)].

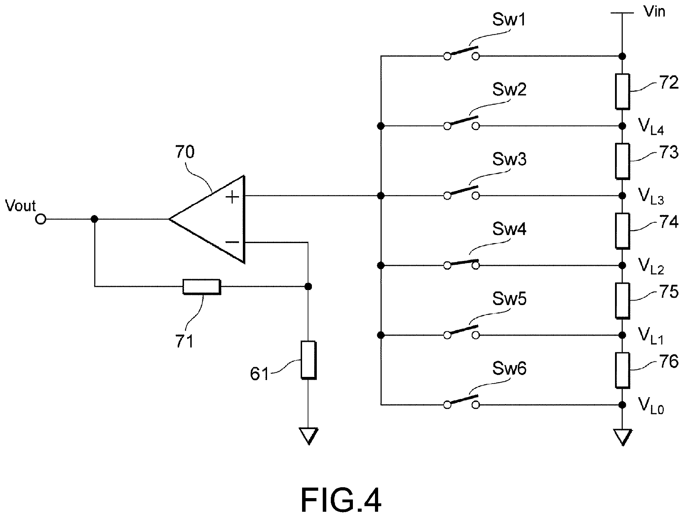

Fig. 4 is a circuit diagram showing an example of the circuit including the heater element 61 of Fig. 2 and Fig. 3. As shown in Fig. 4, the heater element 61 has one end electrically connected to, for example, an inverting input terminal (negative (-) input terminal) of the operational amplifier 70, and the other end connected to the ground. Further, the resistive element 71 is connected in parallel to the operational amplifier 70 between the inverting input terminal and the output terminal thereof. The non-inverting input terminal (positive (+) input terminal) of the operational amplifier 70 is electrically connected to a power source, a point between the resistive element 72 and the resistive element 73 connected in series to each other, a point between the resistive element 73 and the resistive element 74 connected in series to each other, a point between the resistive element 74 and the resistive element 75 connected in series to each other, a point between the resistive element 75 and the resistive element 76 connected in series to each other, or the ground terminal of the resistive element 76, selectively. The resistance values of the resistive elements 72, 73, 74, 75, and 76 are appropriately determined. As a result, when a voltage $V_{in}$ is applied to one end of the resistive element 72, a voltage $V_{L4}$, for example, is generated between the resistive element 73 and the resistive element 72. Similarly, a voltage $V_{L3}$, for example, is generated between the resistive element 74 and the resistive element 73, a voltage $V_{L2}$, for example, is generated between the resistive element 75 and the resistive element 74, and a voltage $V_{L1}$, for example, is generated between the resistive element 76 and the resistive element 75.

A switch Sw1 is provided between the power source and the non-inverting input terminal of the operational amplifier 70, a switch Sw2 is provided between the non-inverting input terminal of the operational amplifier 70 and the point between the resistive element 72 and the resistive element 73, and a switch Sw3 is provided between the non-inverting input terminal of the operational amplifier 70 and the point between the resistive element 73 and the resistive element 74. In addition, a switch Sw4 is provided between the non-inverting input terminal of the operational amplifier 70 and the point between the resistive element 74 and the resistive element 75, a switch Sw5 is provided between the non-inverting input terminal of the operational amplifier 70 and the point between the resistive element 75 and the resistive element 76, and a switch Sw6 is provided between the ground terminal of the resistive element 76 and the non-inverting input terminal of the operational amplifier 70.

If a voltage $V_{in}$ is applied to the non-inverting input terminal of the operational amplifier 70, only the switch Sw1 is turned on (closed) and energized, and the switches Sw2, Sw3, Sw4, Sw5, and Sw6 are turned off (opened) and disconnected. If a voltage $V_{L4}$ is applied to the non-inverting input terminal of the operational amplifier 70, only the switch Sw2 is turned on (closed) and energized, and the switches Sw1, Sw3, Sw4, Sw5, and Sw6 are turned off (opened) and disconnected. If a voltage $V_{L3}$ is applied to the non-inverting input terminal of the operational amplifier 70, only the switch Sw3 is turned on (closed) and energized, and the switches Sw1, Sw2, Sw4, Sw5, and Sw6 are turned off (opened) and disconnected. If a voltage $V_{L2}$ is applied to the non-inverting input terminal of the operational amplifier 70, only the switch Sw4 is turned on (closed) and energized, and the switches Sw1, Sw2, Sw3, Sw5, and Sw6 are turned off (opened) and disconnected. If a voltage $V_{L1}$ is applied to the non-inverting input terminal of the operational amplifier 70, only the switch Sw5 is turned on (closed) and energized, and the switches Sw1, Sw2, Sw3, Sw4, and Sw6 are turned off (opened) and disconnected. If a voltage $V_{L0}$ is applied to the non-inverting input terminal of the operational amplifier 70, only the switch Sw6 is turned on (closed) and energized, and the switches Sw1, Sw2, Sw3, Sw4, and Sw5 are turned off (opened) and disconnected. Therefore, 0 [V] or a voltage of one of five levels may be applied to the non-inverting input terminal of the operational amplifier 70 by turning on and off (closing and opening) the switches Sw1, Sw2, Sw3, Sw4, Sw5, and Sw6. That is, an application voltage which determines the temperature of the heater element 61 can be set in five levels by turning on and off (closing and opening) the switches Sw1, Sw2, Sw3, Sw4, Sw5, and Sw6.

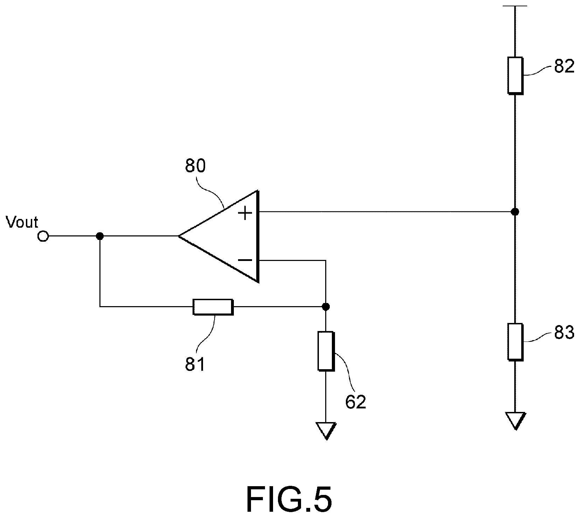

Fig. 5 is a circuit diagram showing an example of the circuit including the first temperature measurement element 62 of Fig. 2 and Fig. 3. As shown in Fig. 5, the first temperature measurement element 62 is electrically connected to, for example, the inverting input terminal (negative (-) input terminal) of the operational amplifier 80 at one end, and is connected to the ground at the other end. In addition, the resistive element 81 is connected in parallel to the operational amplifier 80 between the inverting input terminal and the output terminal thereof. The non-inverting input terminal (positive (+) input terminal) of the operational amplifier 80 is electrically connected to a point between the resistive element 82 and the resistive element 83, which are connected in series. The resistance values of the resistive elements 81, 82, and 83 are determined appropriately. A weak voltage of for example 0.3 [V], which is not enough to generate self-heating, is thus applied to the first temperature measurement element 62.

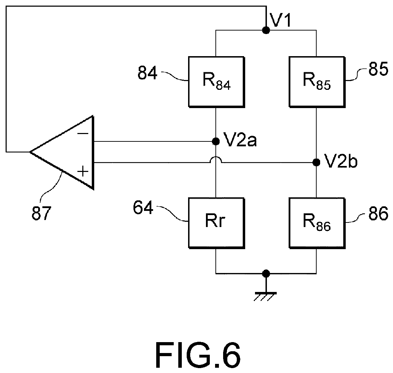

Fig. 6 is a circuit diagram showing an example of the circuit including the heat-retaining element 64 of Fig. 2 and Fig. 3. As shown in Fig. 6, a resistance bridge circuit includes the heat-retaining element 64. The resistance bridge circuit further includes the resistive element 84, the resistive element 85, and the resistive element 86. The resistive element 84 is connected in series to the heat-retaining element 64. The resistive element 85 and the resistive element 86 are connected in parallel to the heat-retaining element 64 and the resistive element 84. Here, the resistance value of the heat-retaining element 64 is Rr. The fixed resistance values of the resistive elements 84, 85, and 86 are $R_{84}$, $R_{85}$, and $R_{86}$, respectively. The operational amplifier 87 is connected to the resistance bridge circuit. The bridge driving voltage $V_1$ is feedback-controlled such that the bridge voltage $V_{2a}$ between the resistive element 84 and the heat-retaining element 64 is the same as the bridge voltage $V_{2b}$ between the resistive element 85 and the resistive element 86. As a result, the resistance value $R_r$ of the heat-retaining element 64 becomes constant, whereby the heat-retaining element 64 generates heat at a constant temperature.

The driving circuit 130 of Fig. 1 is configured to supply driving power, and is connected to the microchip 120 and the controller 150. The driving circuit 130 turns off and on (opens and closes) the above-mentioned switches Sw1, Sw2, Sw3, Sw4, Sw5, and Sw6 based on control signals input from the controller 150. As a result, the driving circuit 130 supplies driving power of a predetermined power (predetermined watt) to the heater element 61 of the microchip 120 of Fig. 2 and Fig. 3. When the driving circuit 130 supplies driving power to the heater element 61, the heater element 61 generates heat at a temperature corresponding to the power (watt) of the driving power. As a result, the heater element 61 is capable of generating heat at a plurality of different temperatures.

The A/D converter 140 of Fig. 1 is configured to convert analog electric signals (hereinafter, arbitrarily referred to as input signals) to digital electric signals (hereinafter, arbitrarily referred to as output signals), and is connected to the microchip 120 and the controller 150. The A/D converter 140 converts an input signal, which is input from the heater element 61 of the microchip 120, to an output signal, and outputs the output signal. Further, the A/D converter 140 converts an input signal, which is input from the first temperature measurement element 62 of the microchip 120, to an output signal, and outputs the output signal. For example, if the A/D converter 140 is a double integrator, the A/D converter 140 outputs a count value as an output signal. Note that the A/D converter 140 may process (e.g., filtering, signal amplification, etc.) an input signal, convert the processed signal to an output signal, and output the output signal.

The controller 150 of Fig. 1 is configured to control the respective blocks of the calorific value measurement system 100. Further, the controller 150 is configured to execute a method of measuring a calorific value (described later) according to the present invention. The controller 150 includes (i.e., functions as) the measurement module 151, the determination module 152, and the calorific value calculation module 153.

The measurement module 151 is configured to measure values of electric signals from the heater element 61 and the first temperature measurement element 62 in contact with gas in the chamber 110.

The determination module 152 is configured to determine if gas in the chamber 110 is a measurement target or not.

The calorific value calculation module 153 is configured to calculate a calorific value of gas in the chamber 110.

Note that the controller 150 includes, for example, a CPU (Central Processing Unit), a GPU (Graphic Processing Unit), memories such as a ROM (Read Only Memory), a RAM (Random Access Memory), and a buffer, various interfaces such as an input interface and an output interface, and a bus connecting them. Meanwhile, a computer (microprocessor) may execute a program to thereby realize functions of the respective blocks of the controller 150. The respective blocks of the controller 150 may thus be realized by hardware, software, or the combination of hardware and software. The present technology is not limited to any one of them.

The storage 160 of Fig. 1 is configured to store data. The controller 150 is connected to the storage 160 such that the controller 150 is capable of accessing the storage 160. The storage 160 stores, for example, measured values of electric signals from the heater element 61 and the first temperature measurement element 62, a calorific value calculation formula of measurement target mixed gas, a threshold (described later), and the like.

A user inputs information by using the input apparatus 170 of Fig. 1. The input apparatus 170 is, for example, a keyboard, a pointing device such as a mouse, or the like. The input apparatus 170 is connected to the controller 150. The input apparatus 170 inputs various kinds of information in the controller 150.

The output apparatus 180 of Fig. 1 is configured to output information for a user. The output apparatus 180 may be an image display apparatus such as a liquid crystal display or a monitor, a printer, or the like. The output apparatus 180 is connected to the controller 150, and outputs information input from the controller 150.

Next, a calorific value calculation formula of mixed gas by the calorific value measurement system 100 will be described.

The resistance value of the heater element 61 shown in Figs. 1 and 2 varies depending on the temperature of the heater element 61. The relationship between the temperature $T_H$ of the heater element 61 and the resistance $R_H$ of the heater element 61 is represented by the following Expression (1):

$$R_H = R_{H\_STD} \times [1 + \alpha_H(T_H - T_{H\_STD}) + \beta_H(T_H - T_{H\_STD})^2] \quad ...(1)$$

where the symbol $T_{H\_STD}$ represents the standard temperature of the heater element 61 and is, for example, 20°C. The symbol $R_{H\_STD}$ represents the resistance value of the heater element 61 which is measured at the standard temperature $T_{H\_STD}$ in advance. The symbol $\alpha_H$ represents a primary resistance-temperature coefficient. The symbol $\beta_H$ represents a secondary resistance-temperature coefficient.

The resistance value $R_H$ of the heater element 61 is calculated from the driving power $P_H$ of the heater element 61 and the on-current IH of the heater element 61 by the following Expression (2):

$$R_H = P_H / I_H^2 \quad ...(2)$$

Alternatively, the resistance value $R_H$ of the heater element 61 is calculated from a voltage $V_H$ applied to the heater element 61 and the on-current $I_H$ of the heater element 61 by the following Expression (3):

$$R_H = V_H / I_H \quad ...(3)$$

Here, the temperature $T_H$ of the heater element 61 is stabilized when the heater element 61 and the atmospheric gas are thermally balanced. The thermally balanced state is a state in which the heat generation of the heater element 61 and the heat loss to the atmospheric gas from the heater element 61 are balanced. As shown in the following Expression (4), by dividing driving power $P_H$ of the heater element 61 in the balanced state by a difference $\Delta T_H$ between the temperature TH of the heater element 61 and the temperature $T_I$ of the atmospheric gas, a radiation coefficient $M_I$ of the atmospheric gas is obtained. The unit of the radiation coefficient $M_I$ is, for example, W/°C.

$$M_I = P_H / (T_H - T_I)$$
$$= P_H / \Delta T_H = (V_H^2 / R_H) / \Delta T_H \quad ...(4)$$

From the above Expression (1), the temperature $T_H$ of the heater element 61 is represented by the following Expression (5):

$$T_H = (1/2\beta_H) \times [-\alpha_H + [\alpha_H^2 - 4\beta_H(1 - R_H/R_{H\_STD})]^{1/2}] + T_{H\_STD} \quad ...(5)$$

Accordingly, the difference $\Delta T_H$ between the temperature $T_H$ of the heater element 61 and the temperature $T_I$ of the atmospheric gas is represented by the following Expression (6):

$$\Delta T_H = (1/2\beta_H) \times [-\alpha_H + [\alpha_H^2 - 4\beta_H(1 - R_H/R_{H\_STD})]^{1/2}] + T_{H\_STD} - I \quad ...(6)$$

The temperature $T_I$ of the atmospheric gas is close to the temperature $T_I$ of the first temperature measurement element 62 which is supplied with power enough not to generate self-eating. The relationship between the temperature $T_I$ of the first temperature measurement element 62 and the resistance $R_I$ of the first temperature measurement element 62 is represented by the following Expression (7):

$$R_I=R_{I\_STD}\times[1+\alpha_I(T_I-T_{I\_STD})+\beta_I(T_I-T_{I\_STD})^2] \ ...(7)$$

where the symbol $T_{H\_STD}$ represents the standard temperature of the first temperature measurement element 62 and is, for example, 20°C. The symbol $R_{I\_STD}$ represents the resistance value of the first temperature measurement element 62 which is measured at the standard temperature $T_{I\_STD}$ in advance. The symbol $\alpha_I$, represents a primary resistance-temperature coefficient. The symbol $\beta_I$ represents a secondary resistance-temperature coefficient.

From the above Expression (7), the temperature $T_I$ of the first temperature measurement element 62 is represented by the following Expression (8):

$$T_I=(1/2\beta_I)\times[-\alpha_I+[\alpha_I{}^2-4\beta_I(1-R_I/R_{I\_STD})]^{1/2}]+T_{I\_STD} \ ...(8)$$

As a result, the heat loss coefficient $M_I$ of the atmosphere gas is given by the formula (9) below.

$$M_I=P_H/\Delta T_H$$
$$=P_H/[(1/2\beta_H)[-\alpha_H+[\alpha_H{}^2-4\beta_H(1-R_H/R_{H\_STD})]^{1/2}]+T_{H\_STD}-(1/2\beta_I)[-\alpha_I+[\alpha_I{}^2-4\beta_I(1-R_I/R_{I\_STD})]^{1/2}]-T_{I\_STD}] \ ...(9)$$

Since the line current $I_H$, driving power $P_H$, or voltage $V_H$ of the heater element 61 can be measured, the resistance value $R_H$ of the heater element 61 can be calculated from the above formula (2) or (3). Likewise, the resistance value $R_I$ of the first temperature measurement element 62 can also be calculated. Therefore, the heat loss coefficient $M_I$ of the atmosphere gas can be calculated from the formula (9) using the microchip 8.

Since the heat-retaining element 64 maintains the temperature of the substrate 60 to be constant, the temperature of the atmosphere gas in the vicinity of the microchip 8 before the heater element 61 generates heat is approximate to the constant temperature of the substrate 60. Therefore, a variation in the temperature of the atmosphere gas before the heater element 61 generates heat is prevented. Since the heater element 61 heats the atmosphere gas whose temperature variation has been prevented, it is possible to calculate the radiation coefficient $M_I$ with high accuracy.

It is assumed that the atmosphere gas is a mixed gas and the mixed gas includes four kinds of gas components A, B, C, and D. The sum of the volume ratio $V_A$ of the gas A, the volume ratio $V_B$ of the gas B, the volume ratio $V_C$ of the gas C, and the volume ratio $V_D$ of the gas D is 1, as represented by the following Expression (10):

$$V_A+V_B+V_C+V_D=1 \ ...(10)$$

When the calorific value of the gas A per unit volume is $K_A$, the calorific value of the gas B per unit volume is $K_B$, the calorific value of the gas C per unit volume is $K_C$, and the calorific value of the gas D per unit volume is $K_D$, the calorific value Q of the mixed gas per unit volume is the sum of the values obtained by multiplying the volume ratios of the gas components by the calorific values of the gas components per unit volume. Therefore, the calorific value Q of the mixed gas per unit volume is represented by the following Expression (11):

$$Q=K_A\times V_A+K_B\times V_B+K_C\times V_C+K_D\times V_D \ ... \ (11)$$

The unit of the calorific value per unit volume is, for example, $MJ/m^3$.

When the radiation coefficient of the gas A is $M_A$, the radiation coefficient of the gas B is $M_B$, the radiation coefficient of the gas C is $M_C$, and the radiation coefficient of the gas D is $M_D$, the radiation coefficient $M_I$ of the mixed gas is the sum of the values obtained by multiplying the volume ratios of the gas components by the radiation coefficients of the gas components. Therefore, the radiation coefficient $M_I$ of the mixed gas is represented by the following Expression (12):

$$M_I=M_A\times V_A+M_B\times V_B+M_C\times V_C+M_D\times V_D \ ... \ (12)$$

Further, since the radiation coefficient of gas depends on the heat generating temperature $T_H$ of the heater element 61, the radiation coefficient $M_I$ of the mixed gas is represented as a function of the temperature $T_H$ of the heater element 61 by the following Expression (13):

$$M_I = M_A x V_A + M_B x V_B + M_C x V_C + M_D x V_D \ ...(13)$$

As a result, the heat loss coefficient $M_{I1}$ ($T_{H1}$) of the mixed gas is given by the formula (14) below when the temperature of the heater element 61 is $T_{H1}$. In addition, the heat loss coefficient $M_{I2}$ ($T_{H2}$) of the mixed gas is given by the formula (15) below when the temperature of the heater element 61 is $T_{H2}$, and the heat loss coefficient $M_{I3}$ ($T_{H3}$) of the mixed gas is given by the formula (16) below when the temperature of the heater element 61 is $T_{H3}$, where the temperatures $T_{H1}$, $T_{H2}$, and $T_{H3}$ of the heater element 61 are different from each other.

$$M_{I1}(T_{H1}) = M_A(T_{H1}) x V_A + M_B(T_{H1}) x V_B + M_C(T_{H1}) x V_C + M_D(T_{H1}) x V_D \ ...(14)$$

$$M_{I2}(T_{H2}) = M_A(T_{H2}) x V_A + M_B(T_{H2}) x V_B + M_C(T_{H2}) x V_C + M_D(T_{H2}) x V_D \ ...(15)$$

$$M_{I3}(T_{H3}) = M_A(T_{H3}) x V_A + M_B(T_{H3}) x V_B + M_C(T_{H3}) x V_C + M_D(T_{H3}) x V_D \ ...(16)$$

Here, when the radiation coefficients $M_A$ ($T_H$), $M_B$ ($T_H$), $M_C$ ($T_H$), and $M_D$ ($T_H$) of the gas components relative to the temperature $T_H$ of the heater element 61 have nonlinearity, the above Expressions (14) to (16) have a linear independent relationship. In addition, even when the radiation coefficients $M_A$ ($T_H$), $M_B$ ($T_H$), $M_C$ ($T_H$), and $M_D$ ($T_H$) of the gas components relative to the temperature $T_H$ of the heater element 61 have linearity, when the change ratios of the radiation coefficients $M_A$ ($T_H$), $M_B$ ($T_H$), $M_C$ ($T_H$), and $M_D$ ($T_H$) of the gas components relative to the temperature TH of the heater element 61 are different from each other, the above Expressions (14) to (16) have a linear independent relationship. Further, when Expressions (14) to (16) have a linear independent relationship, Expressions (10) and (14) to (16) have a linear independent relationship.

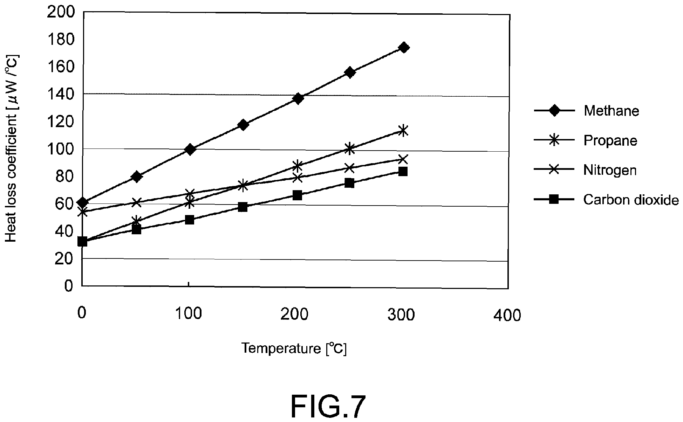

Fig. 7 is a graph showing an example of the relationship between temperature of the heater element 61 of Fig. 2 and Fig. 3 and heat loss coefficients of mixed gas. Note that the graph of Fig. 7 shows the heat loss coefficients of methane ($CH_4$), propane ($C_3H_8$), nitrogen ($N_2$), and carbon dioxide ($CO_2$) which are gas components of natural gas. As shown in Fig. 7, the heat loss coefficient of each gas component (methane ($CH_4$), propane ($C_3H_8$), nitrogen ($N_2$), or carbon dioxide ($CO_2$) is linear with respect to the temperature of the heater element 61. Meanwhile, the change rate of the heat loss coefficient of each gas component (methane ($CH_4$), propane ($C_3H_8$), nitrogen ($N_2$), or carbon dioxide ($CO_2$)) to the temperature of the heater element 61 is different from each other. Because of this, if the gas components of mixed gas are methane ($CH_4$), propane ($C_3H_8$), nitrogen ($N_2$), and carbon dioxide ($CO_2$) each of the formula (14) to the formula (16) has a linear independent relationship.

Values of the heat loss coefficients $M_A$ ($T_{H1}$), $M_B$ ($T_{H1}$), $M_C$ ($T_{H1}$), $M_D$ ($T_{H1}$), $M_A$ ($T_{H2}$), $M_B$ ($T_{H2}$), $M_C$ ($T_{H2}$), $M_D$ ($T_{H2}$), $M_A$ ($T_{H3}$), $M_B$ ($T_{H3}$), $M_C$ ($T_{H3}$), and $M_D$ ($T_{H3}$) of the respective gas components in the formula (14) to (16) can be obtained in advance through measurement or the like. Therefore, if solving the simultaneous equations of the formula (10) and (14) to (16), each of the volume ratio $V_A$ of the gas A, the volume ratio $V_B$ of the gas B, the volume ratio $V_C$ of the gas C, and the volume ratio $V_D$ of the gas D is given as a function of the heat loss coefficients $M_{I1}$ ($T_{H1}$), $M_{I2}$ ($T_{H2}$), and $M_{I3}$ ($T_{H3}$) of the mixed gas as shown in the formula (17) to (20) below, where, in the formula (17) to the formula (20), n is a positive integer and $f_n$ represents a function.

$$V_A = f_1[M_{I1}(T_{H1}), M_{I2}(T_{H2}), M_{I3}(T_{H3})] \ ...(17)$$

$$V_B = f_2[M_{I1}(T_{H1}), M_{I2}(T_{H2}), M_{I3}(T_{H3})] \ ...(18)$$

$$V_C = f_3[M_{I1}(T_{H1}), M_{I2}(T_{H2}), M_{I3}(T_{H3})] \ ...(19)$$

$$V_D=f_4[M_{I1}(T_{H1}), M_{I2}(T_{H2}), M_{I3}(T_{H3})] \;...(20)$$

Here, the formula (21) below can be obtained by substituting the formula (17) to (20) into the formula (11).

$$Q=K_A \times V_A+K_B \times V_B+K_C \times V_C+K_D \times V_D$$
$$=K_A \times f_1[M_{I1}(T_{H1}), M_{I2}(T_{H2}), M_{I3}(T_{H3})]$$
$$+K_B \times f_2[M_{I1}(T_{H1}), M_{I2}(T_{H2}), M_{I3}(T_{H3})]$$
$$+K_C \times f_3[M_{I1}(T_{H1}), M_{I2}(T_{H2}), M_{I3}(T_{H3})]$$
$$+K_D \times f_4[M_{I1}(T_{H1}), M_{I2}(T_{H2}), M_{I3}(T_{H3})] \;...(21)$$

As shown in the formula (21), the calorific value Q per unit volume of the mixed gas is given by a formula including the heat loss coefficients $M_{I1}(T_{H1})$, $M_{I2}(T_{H2})$, and $M_{I3}(T_{H3})$ of the mixed gas when the temperatures of the heater element 61 are $T_{H1}$, $T_{H2}$, and $T_{H3}$ as variables. As a result, the calorific value Q of the mixed gas is given by the formula (22) below, where g represents a function.

$$Q=g[M_{I1}(T_{H1}), M_{I2}(T_{H2}), M_{I3}(T_{H3})] \;...(22)$$

As a result, if the above formula (22) is obtained in advance for the mixed gas made up of the gas A, the gas B, the gas C, and the gas D, the calorific value Q per unit volume of the inspection subject mixed gas of which the volume ratio $V_A$ of the gas A, the volume ratio $V_B$ of the gas B, the volume ratio $V_C$ of the gas C, and the volume ratio $V_D$ of the gas D are unknown can be easily calculated.

In detail, by measuring the heat loss coefficients $M_{I1}(T_{H1})$, $M_{I2}(T_{H2})$, and $M_{I3}(T_{H3})$ of the inspection subject mixed gas when the temperatures of the heater element 61 are $T_{H1}$, $T_{H2}$, and $T_{H3}$ and then substituting them into the formula (22), it becomes possible to obtain a unique calorific value Q of the inspection subject mixed gas.

In addition, the heat loss coefficient $M_I$ of the mixed gas is dependent on the resistance value $R_H$ of the heater element 61 and the resistance value $R_I$ of the first temperature measurement element 62 as shown in the formula (9). Therefore, the inventors of the invention found that, as shown in the formula (23) below, the calorific value Q per unit volume of the mixed gas is also given by a formula including the resistance values $R_{H1}(T_{H1})$, $R_{H2}(T_{H2})$, and $R_{H3}(T_{H3})$ of the heater element 61 when the temperatures of the heater element 61 are $T_{H1}$, $T_{H2}$, and $T_{H3}$, and the resistance value $R_I$ of the first temperature measurement element 62 that comes into contact with the mixed gas, as variables.

$$Q=g[R_{H1}(T_{H1}), R_{H2}(T_{H2}), R_{H3}(T_{H3}), R_I] \;...(23)$$

Therefore, also by measuring the resistance values $R_{H1}(T_{H1})$, $R_{H2}(T_{H2})$, and $R_{H3}(T_{H3})$ of the heater element 61 that comes into the inspection subject mixed gas when the temperatures of the heater element 61 are $T_{H1}$, $T_{H2}$ and $T_{H3}$, and the resistance value $R_I$ of the first temperature measurement element 62 that comes into contact with the inspection subject mixed gas and then substituting them into the formula (23), it becomes possible to obtain a unique calorific value Q of the inspection subject mixed gas.

In addition, as shown in the formula (24) below, the calorific value Q per unit volume of the mixed gas is also given by a formula including the line currents $I_{H1}(T_{H1})$, $I_{H2}(T_{H2})$, and $I_{H3}(T_{H3})$ of the heater element 61 when the temperatures of the heater element 61 are $T_{H1}$, $T_{H2}$, and $T_{H3}$, and the line current $I_I$ of the first temperature measurement element 62 that comes into contact with the mixed gas as variables.

$$Q=g[I_{H1}(T_{H1}), I_{H2}(T_{H2}), I_{H3}(T_{H3}), I_I] \;...(24)$$

Alternately, as shown in the formula (25) below, the calorific value Q per unit volume of the mixed gas is also given by a formula including the voltages $V_{H1}(T_{H1})$, $V_{H2}(T_{H2})$, and $V_{H3}(T_{H3})$ applied to the heater element 61 when the temperatures of the heater element 61 are $T_{H1}$, $T_{H2}$, and $T_{H3}$, and the voltage $V_I$ applied to the first temperature measurement element 62 that comes into contact with the mixed gas, as variables.

$$Q=g[V_{H1}(T_{H1}), V_{H2}(T_{H2}), V_{H3}(T_{H3}), V_1] \;...(25)$$

In addition, alternatively, as shown in the formula (26) below, the calorific value Q per unit volume of the mixed gas is also given by a formula including the output signals $AD_{H1}$ ($TH_1$), $AD_{H2}$ ($T_{H2}$), and $AD_{H3}$ ($T_{H3}$) of the analog-digital converter (hereinafter referred to as "A/D converter"), which is connected to the heater element 61, when the temperatures of the heater element 61 are $T_{HI,}$ $T_{H2,}$ and $T_{H3}$, and the output signal $AD_I$ of the A/D converter, which is connected to the first temperature measurement element 62 that comes into contact with the mixed gas, as variables.

$$Q=g[AD_{I1}(T_{H1}), AD_{I2}(T_{H2}), AD_{I3}(T_{H3}), AD_1] \;...(26)$$

As a result, as shown in the formula (27) below, the calorific value Q per unit volume of the mixed gas is also given by a formula including the electric signals $S_{H1}$ ($T_{H1}$), $S_{H2}$ ($T_{H2}$), and $S_{H3}$ ($T_{H3}$) from the heater element 61 when the temperatures of the heater element 61 are $T_{H1}$, $T_{H2}$, and $T_{H3}$, and the electric signal $S_I$ from the first temperature measurement element 62 that comes into contact with the mixed gas, as variables.

$$Q=g[S_{H1}(T_{H1}), S_{H2}(T_{H2}), S_{H3}(T_{H3}), S_1] \;...(27)$$

Further, if the temperature of mixed gas is constant, the electric signal $S_I$ from the first temperature measurement element 62 is a constant number. In this case, as shown in the following formula (28), the calorific value Q per unit volume of mixed gas is also obtained by a formula where only the electric signals $S_{H1}(T_{H1})$, $S_{H2}(T_{H2})$, and $S_{H3}(T_{H3})$ from the heater element 61 are variables.

$$Q = g[S_{H1}(T_{H1}), S_{H2}(T_{H2}), S_{H3}(T_{H3})]...(28)$$

Meanwhile, the gas components of the mixed gas are not limited to 4 kinds. For example, when the mixed gas is made up of n kinds of gas components, firstly, as shown in the formula (29), a formula including the electric signals $S_{H1}$ ($T_{H1}$), $S_{H2}$ ($T_{H2}$), $S_{H3}$ ($T_{H3}$), ... , $S_{Hn-1}$ ($T_{Hn-1}$) from the heater element 61 at the heating temperatures $T_{H1}$, $T_{H2}$, $T_{H3}$, ..., $T_{Hn-1}$ of at least n-1 kinds, and the electric signal SI from the first temperature measurement element 62 that comes into contact with the mixed gas, which are given in the formula (28) below, as variables, is obtained in advance. Then, the values of the electric signals $S_{H1}$ ($T_{H1}$), $S_{H2}$ ($T_{H2}$), $S_{H3}$ ($T_{H3}$), ..., $S_{Hn-1}$ ($T_{Hn-1}$) from the heater element 61 that comes into contact with the inspection subject mixed gas of which each of the volume ratios of n kinds of gas components are unknown at the heating temperatures $T_{H1}$, $T_{H2}$, $T_{H3}$, ..., $T_{Hn-1}$ of n-1 kinds, and the value of the electric signal SI from the first temperature measurement element 62 that comes into contact with the inspection subject mixed gas, are measured and substituted into the formula (29), whereby it becomes possible to obtain the unique calorific value Q per unit volume of the inspection subject mixed gas.

$$Q=g[S_{H1}(T_{H1}), SD_{H2}(T_{H2}), SD_{H3}(T_{H3}), ..., S_{Hn-1} (T_{Hn-1}) ,S_1] \;...(29)$$

However, if the mixed gas includes alkane ($C_jH_{2j+2}$), in which j is a natural number, other than methane ($CH_4$) and propane ($C_3H_8$) as the gas components in addition to methane ($CH_4$) and propane ($C_3H_8$), the calculation of the formula (28) is not affected even when alkane ($C_jH_{2j+2}$) other than methane ($CH_4$) and propane ($C_3H_8$) is considered as a mixture of methane ($CH_4$) and propane ($C_3H_8$). For example, it makes no change to consider, for example, ethane ($C_2H_6$), butane ($C_4H_{10}$), pentane ($C_5H_{12}$), and hexane ($C_6H_{14}$) as a mixture of methane ($CH_4$) and propane ($C_3H_8$) multiplied by a predetermined coefficient respectively and then calculate the formula (29) as shown in the formula (30) to (33).

$$C_2H_6 = 0.5\, CH_4 + 0.5\, C_3H_8 \cdots \qquad (30)$$

$$C_4H_{10} = -0.5\, CH_4 + 1.5\, C_3H_8 \cdots \qquad (31)$$

$$C_5H_{12} = -1.0\, CH_4 + 2.0\, C_3H_8 \cdots \qquad (32)$$

$$C_6H_{14} = -1.5 \, CH_4 + 2.5 \, C_3H_8 \cdots \qquad (33)$$

As a result, when the mixed gas made up of n kinds of gas components includes z kinds of alkane ($C_jH_{2j+2}$) other than methane ($CH_4$) and propane ($C_3H_8$) in addition to methane ($CH_4$) and propane ($C_3H_8$) as the gas components, in which z is a natural number, it is allowed to obtain a formula including the electric signals $S_H$ from the heater element 61 at a minimum of n-z-1 kinds of the heating temperatures and the electric signal $S_I$ from the first temperature measurement element 62 as variables.

When the kind of gas components in the mixed gas used to calculate Expression (29) is equal to the kind of gas components in a mixed gas to be examined whose calorific value Q per unit volume is unknown, Expression (29) may be used to calculate the calorific value Q of the mixed gas to be examined. When the mixed gas to be examined includes gas components that are smaller than n kinds of gas components and gas components smaller than n kinds of gas components are included in the mixed gas used to calculate Expression (29), Expression (29) can be used. For example, when the mixed gas used to calculate Expression (29) includes four kinds of gas components, that is, methane ($CH_4$), propane ($C_3H_8$), nitrogen ($N_2$), and carbon dioxide ($CO_2$) and the mixed gas to be examined does not include nitrogen ($N_2$), but includes only three kinds of gas components, that is, methane ($CH_4$), propane ($C_3H_8$), and carbon dioxide ($CO_2$), Expression (29) can also be used to calculate the calorific value Q of the mixed gas to be examined.

When the mixed gas used to calculate Expression (28) includes as gas components methane ($CH_4$) and propane ($C_3H_8$), Expression (28) can be used even when the mixed gas to be examined includes alkane ($C_jH_{2j+2}$) which is not included in the mixed gas used to calculate Expression (28). This is because regarding alkane ($C_jH_{2j+2}$) other than methane ($CH_4$) and propane ($C_3H_8$) as a mixture of methane ($CH_4$) and propane ($C_3H_8$) does not affect the calculation of the calorific value Q per unit volume by Expression (28), as described above.

Next, an example of the configuration of a system configured to create a calorific value calculation formula of mixed gas will be described. The calorific value measurement system 100 uses the calorific value calculation formula.

Here, four kinds of mixed gas (hereinafter, arbitrarily referred to as first to fourth sample mixed gases) having different calorific values Q, respectively, are suppositionally prepared as samples in order to create a calorific value calculation formula of measurement target mixed gas. For example, the first to fourth sample mixed gases are natural gas having different calorific values. Each of the first to fourth sample mixed gases includes, for example, four kinds of gas components, that is, methane ($CH_4$), propane ($C_3H_8$), nitrogen ($N_2$), and carbon dioxide ($CO_2$) at different rates (percentages).

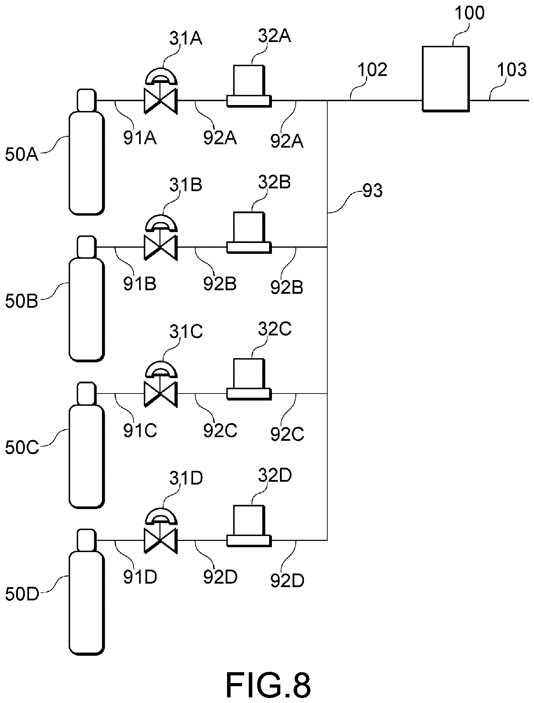

Fig. 8 is a diagram schematically showing the configuration of a calorific value calculation formula creation system. The system includes the calorific value measurement system 100 of Fig. 1. As shown in Fig. 8, the first gas cylinder 50A storing a first sample mixed gas, the second gas cylinder 50B storing a second sample mixed gas, the third gas cylinder 50C storing a third sample mixed gas, and the fourth gas cylinder 50D storing a fourth sample mixed gas are prepared. The first gas pressure adjuster 31 A is configured to adjust the pressure of the first sample mixed gas, and is connected to the first gas cylinder 50A via the flow path 91 A. In addition, the first flow rate controller 32A is connected to the first gas pressure adjuster 31 A through the flow path 92A. The first flow rate controller 32A is configured to control the flow rate of the first sample mixed gas transferred to the calorific value measurement system 100 through the flow path 92A and a flow path 102.

The second gas pressure adjuster 31 B is configured to adjust the pressure of the second sample mixed gas, and is connected to the second gas cylinder 50B via the flow path 91 B. In addition, the second flow rate controller 32B is connected to the second gas pressure adjuster 31 B through the flow path 92B. The second flow rate controller 32B is configured to control the flow rate of the second sample mixed gas transferred to the calorific value measurement system 100 through the flow paths 92B, 93, and 102.

The third gas pressure adjuster 31C is configured to adjust the pressure of the third sample mixed gas, and is connected to the third gas cylinder 50C via the flow path 91C. In addition, the third flow rate controller 32C is connected to the third gas pressure adjuster 31C through the flow path 92C. The third flow rate controller 32C is configured to control the flow rate of the third sample mixed gas transferred to the calorific value measurement system 100 through the flow paths 92C, 93, and 102.

The fourth gas pressure adjuster 31 D is configured to adjust the pressure of the fourth sample mixed gas, and is connected to the fourth gas cylinder 50D via the flow path 91 D. In addition, the fourth flow rate controller 32D is connected to the fourth gas pressure adjuster 31 D through the flow path 92D. The fourth flow rate controller 32D is configured to control the flow rate of the fourth sample mixed gas transferred to the calorific value measurement system 100 through the flow paths 92D, 93, and 102.

Next, an example of the procedure of creating a calorific value calculation formula of mixed gas by the calorific value measurement system 100 will be described.

First, the valve of the first flow rate controller 32A is opened while the valves of the second to fourth flow rate controllers 32B, 32C, and 32D are closed. The first sample mixed gas is introduced in the chamber 110 of Fig. 1, and the chamber 110 is filled with the first sample mixed gas. A weak voltage, which is not enough to generate self-heating, is applied to the first temperature measurement element 62 of the microchip 120 of Fig. 2 and Fig. 3. The measurement module 151 of Fig. 1 measures the value of the electric signal $S_I$, which depends on the temperature of the first sample mixed gas. Further, the measurement module 151 writes the measured value of the electric signal $S_I$ from the first temperature measurement element 62 (hereinafter, arbitrarily referred to as measured value) in the storage 160. The storage 160 stores the measured value.

Next, the measurement module 151 outputs a control signal to the driving circuit 130 of Fig. 1. In response to the control signal input from the measurement module 151, the driving circuit 130 supplies driving powers $P_{H1}$, $P_{H2}$, and $P_{H3}$ to the heater element 61 of the microchip 120 of Fig. 2 and Fig. 3 in order. The heater element 61 in contact with the first sample mixed gas generates heat at the temperatures $T_{H1}$, $T_{H2}$, and $T_{H3}$ corresponding to the driving powers $P_{H1}$, $P_{H2}$, and $P_{H3}$, respectively. When driving powers $P_{H1}$, $P_{H2}$, and $P_{H3}$ are supplied, the heater element 61 generates heat, for example, at a temperature $T_{H1}$ of 100[deg.]C, a temperature $T_{H2}$ of 150[deg.]C, and a temperature $T_{H3}$ of 200[deg.]C, respectively. Further, the measurement module 151 measures the value of the electric signal $S_{H1}(T_{H1})$ at the temperature $T_{H1}$, the value of the electric signal $S_{H2}(T_{H2})$ at the temperature $T_{H2}$, and the value of the electric signal $S_{H3}(T_{H3})$ at the temperature $T_{H3}$. Further, the measurement module 151 writes the measured values of the electric signals $S_{H1}(T_{H1})$, $S_{H2}(T_{H2})$, and $S_{H3}(T_{H3})$ from the heater element 61 (hereinafter, arbitrarily referred to as measured values) in the storage 160. The storage 160 stores the measured values. After the values of the electric signals $S_{H1}(T_{H1})$, $S_{H2}(T_{H2})$, and $S_{H3}(T_{H3})$ from the heater element 61 are measured, the first sample mixed gas is exhausted from the chamber 110 and removed.

The similar procedure applies to the second to fourth sample mixed gases.

That is, after the first sample mixed gas is removed from the chamber 110, the valve of the second flow rate controller 32B is opened while the valves of the first, third, and fourth flow rate controllers 32A, 32C, and 32D are closed. The second sample mixed gas is introduced in the chamber 110 of Fig. 1, and the chamber 110 is filled with the second sample mixed gas. A weak voltage, which is not enough to generate self-heating, is applied to the first temperature measurement element 62 of the microchip 120 of Fig. 2 and Fig. 3. The measurement module 151 of Fig. 1 measures the value of the electric signal $S_I$ from the first temperature measurement element 62, which depends on the temperature of the second sample mixed gas. Further, the measurement module 151 writes the measured value of the electric signal $S_I$ from the first temperature measurement element 62 (hereinafter, arbitrarily referred to as measured value) in the storage 160. The storage 160 stores the measured value.

Next, the measurement module 151 outputs a control signal to the driving circuit 130 of Fig. 1. In response to the control signal input from the measurement module 151, the driving circuit 130 supplies driving powers $P_{H1}$, $P_{H2}$, and $P_{H3}$ to the heater element 61 of the microchip 120 of Fig. 2 and Fig. 3 in order. The heater element 61 in contact with the second sample mixed gas generates heat at the temperatures $T_{H1}$, $T_{H2}$, and $T_{H3}$ corresponding to the driving powers $P_{H1}$, $P_{H2}$, and $P_{H3}$, respectively. When driving powers $P_{H1}$, $P_{H2}$, and $P_{H3}$ are supplied, the heater element 61 generates heat, for example, at a temperature $T_{H1}$ of 100[deg.]C, a temperature $T_{H2}$ of 150[deg.]C, and a temperature $T_{H3}$ of 200[deg.]C, respectively. Further, the measurement module 151 measures the value of the electric signal $S_{H1}(T_{H1})$ at the temperature $T_{H1}$, the value of the electric signal $S_{H2}(T_{H2})$ at the temperature $T_{H2}$, and the value of the electric signal $S_{H3}(T_{H3})$ at the temperature $T_{H3}$. Further, the measurement module 151 writes the measured values of the electric signals $S_{H1}(T_{H1})$, $S_{H2}(T_{H2})$, and $S_{H3}(T_{H3})$ from the heater element 61 (hereinafter, arbitrarily referred to as measured values) in the storage 160. The storage 160 stores the measured values. After the values of the electric signals $S_{H1}(T_{H1})$, $S_{H2}(T_{H2})$, and $S_{H3}(T_{H3})$ from the heater element 61 are measured, the second sample mixed gas is exhausted from the chamber 110 and removed.

After the second sample mixed gas is removed from the chamber 110, the valve of the third flow rate controller 32C is opened while the valves of the first, second, and fourth flow rate controllers 32A, 32B, and 32D are closed. The third sample mixed gas is introduced in the chamber 110 of Fig. 1, and the chamber 110 is filled with the third sample mixed gas. A weak voltage, which is not enough to generate self-heating, is applied to the first temperature measurement element 62 of the microchip 120 of Fig. 2 and Fig. 3. The measurement module 151 of Fig. 1 measures the value of the electric signal $S_I$ from the first temperature measurement element 62, which depends on the temperature of the third sample mixed gas. Further, the measurement module 151 writes the measured value of the electric signal $S_1$ from the first temperature measurement element 62 (hereinafter, arbitrarily referred to as measured value) in the storage 160. The storage 160 stores the measured value.

Next, the measurement module 151 outputs a control signal to the driving circuit 130 of Fig. 1. In response to the control signal input from the measurement module 151, the driving circuit 130 supplies driving powers $P_{H1}$, $P_{H2}$, and $P_{H3}$ to the heater element 61 of the microchip 120 of Fig. 2 and Fig. 3 in order. The heater element 61 in contact with the third sample mixed gas generates heat at the temperatures $T_{H1}$, $T_{H2}$, and $T_{H3}$ corresponding to the driving powers $P_{H1}$, $P_{H2}$, and $P_{H3}$, respectively. When driving powers $P_{H1}$, $P_{H2}$, and $P_{H3}$ are supplied, the heater element 61 generates heat, for example, at a temperature $T_{H1}$ of 100[deg.]C, a temperature $T_{H2}$ of 150[deg.]C, and a temperature $T_{H3}$ of 200[deg.]C, respectively. Further, the measurement module 151 measures the value of the electric signal $S_{H1}(T_{H1})$ at the temperature $T_{H1}$, the value of the electric signal $S_{H2}(T_{H2})$ at the temperature $T_{H2}$, and the value of the electric signal

$S_{H3}(T_{H3})$ at the temperature $T_{H3}$. Further, the measurement module 151 writes the measured values of the electric signals $S_{H1}(T_{H1})$, $S_{H2}(T_{H2})$, and $S_{H3}(T_{H3})$ from the heater element 61 (hereinafter, arbitrarily referred to as measured values) in the storage 160. The storage 160 stores the measured values. After the values of the electric signals $S_{H1}(T_{H1})$, $S_{H2}(T_{H2})$, and $S_{H3}(T_{H3})$ from the heater element 61 are measured, the third sample mixed gas is exhausted from the chamber 110 and removed.

After the third sample mixed gas is removed from the chamber 110, the valve of the fourth flow rate controller 32D is opened while the valves of the first to third flow rate controllers 32A, 32B, and 32C are closed. The fourth sample mixed gas is introduced in the chamber 110 of Fig. 1, and the chamber 110 is filled with the fourth sample mixed gas. A weak voltage, which is not enough to generate self-heating, is applied to the first temperature measurement element 62 of the microchip 120 of Fig. 2 and Fig. 3. The measurement module 151 of Fig. 1 measures the value of the electric signal $S_I$ from the first temperature measurement element 62, which depends on the temperature of the fourth sample mixed gas. Further, the measurement module 151 writes the measured value of the electric signal $S_I$ from the first temperature measurement element 62 (hereinafter, arbitrarily referred to as measured value) in the storage 160. The storage 160 stores the measured value.

Next, the measurement module 151 outputs a control signal to the driving circuit 130 of Fig. 1. In response to the control signal input from the measurement module 151, the driving circuit 130 supplies driving powers $P_{H1}$, $P_{H2}$, and $P_{H3}$ to the heater element 61 of the microchip 120 of Fig. 2 and Fig. 3 in order. The heater element 61 in contact with the fourth sample mixed gas generates heat at the temperatures $T_{H1}$, $T_{H2}$, and $T_{H3}$ corresponding to the driving powers $P_{H1}$, $P_{H2}$, and $P_{H3}$, respectively. When driving powers $P_{H1}$, $P_{H2}$, and $P_{H3}$ are supplied, the heater element 61 generates heat, for example, at a temperature $T_{H1}$ of 100[deg.]C, a temperature $T_{H2}$ of 150[deg.]C, and a temperature $T_{H3}$ of 200[deg.]C, respectively. Further, the measurement module 151 measures the value of the electric signal $S_{H1}(T_{H1})$ at the temperature $T_{H1}$, the value of the electric signal $S_{H2}(T_{H2})$ at the temperature $T_{H2}$, and the value of the electric signal $S_{H3}(T_{H3})$ at the temperature $T_{H3}$. Further, the measurement module 151 writes the measured values of the electric signals $S_{H1}(T_{H1})$, $S_{H2}(T_{H2})$, and $S_{H3}(T_{H3})$ from the heater element 61 (hereinafter, arbitrarily referred to as measured values) in the storage 160. The storage 160 stores the measured values. After the values of the electric signals $S_{H1}(T_{H1})$, $S_{H2}(T_{H2})$, and $S_{H3}(T_{H3})$ from the heater element 61 are measured, the fourth sample mixed gas is exhausted from the chamber 110 and removed.

Here, if each of the first to fourth sample mixed gases includes n kinds of gas components, the measurement module 151 and the driving circuit 130 cause the heater element 61 of the microchip 120 to generate heat at at least n-1 different temperatures. However, as described above, alkane ($C_jH_{2j+2}$) other than methane ($CH_4$) and propane ($C_3H_8$) can be considered as a mixture of methane ($CH_4$) and propane ($C_3H_8$). That is, if each of the first to fourth sample mixed gases made up of n kinds of gas components includes z kinds of alkane ($C_jH_{2j+2}$) in addition to methane ($CH_4$) and propane ($C_3H_8$) as the gas components, where z is a natural number, the measurement module 151 and the driving circuit 130 causes the heater element 61 to generate heat at at least n-z-1 different temperatures.

Note that the electric signal $S_I$ from the first temperature measurement element 62 may be any one of the resistance value $R_I$ of the first temperature measurement element 62, the line current $I_I$ of the first temperature measurement element 62, the voltage $V_I$ applied to the first temperature measurement element 62, and the signal $AD_I$ output from the A/D converter 140 connected to the first temperature measurement element 62. Similarly, the electric signal $S_H$ from the heater element 61 may be any one of the resistance value $R_H$ of the heater element 61, the line current $I_H$ of the heater element 61, the voltage $V_H$ applied to the heater element 61, and the signal $AD_H$ output from the A/D converter 140 connected to the heater element 61.

Meanwhile, for example, the input apparatus 170 inputs a known calorific value Q of the first sample mixed gas, a known calorific value Q of the second sample mixed gas, a known calorific value Q of the third sample mixed gas, and a known calorific value Q of the fourth sample mixed gas in the controller 150 of Fig. 1.

Further, the controller 150 reads a plurality of measured values of the electric signals $S_I$ from the first temperature measurement element 62 and a plurality of measured values of the electric signals $S_H$ from the heater element 61 from the storage 160. The controller 150 calculates a calorific value calculation formula by multivariate analysis based on the plurality of read measured values of the electric signals $S_I$ from the first temperature measurement element 62, the plurality of read measured values of the electric signals $S_H$ from the heater element 61, and the input calorific values Q of the first to fourth sample mixed gases. As a result, the controller 150 creates a calorific value calculation formula, where the electric signal $S_I$ from the first temperature measurement element 62 and the electric signals $S_{H1}(T_{H1})$, $S_{H2}(T_{H2})$, and $S_{H3}(T_{H3})$ from the heater element 61 are independent variables, and the calorific value Q of the mixed gas is a dependent variable. The controller 150 writes the created calorific value calculation formula in the storage 160, and the storage 160 stores the created calorific value calculation formula.

The "multivariate analysis" includes a support vector regression and multiple regression analysis disclosed in A.J. Smola and B. Scholkopf, "A Tutorial on Support Vector Regression" (NeuroCOLT Technical Report (NC-TR-98-030), 1998) and Fuzzy Qualification Theory of Second Order disclosed in JP-A-5-141999.

According to the example of this embodiment, a calorific value calculation formula is created where the electric signal from the first temperature measurement element 62 and the electric signals from the heater element 61 at a plurality of different temperatures are independent variables, and the calorific value Q of the mixed gas is a dependent variable. However, this embodiment is not limited to this example.

For example, as described with reference to the above-mentioned formula (22), the calorific value Q of the mixed gas is given by a formula including, as variables, the heat loss coefficients $M_{I1}(T_{H1})$, $M_{I2}(T_{H2})$, and $M_{I3}(T_{H3})$ of the mixed gas, where $T_{H1}$, $T_{H2}$, and $T_{H3}$ are the temperatures of the heater element 61, respectively. Because of this, the controller 150 of Fig. 1 may create a calorific value calculation formula where heat loss coefficients of mixed gas at a plurality of different temperatures of the heater element 61 are independent variables, and the calorific value Q is a dependent variable. The controller 150 may write the created calorific value calculation formula in the storage 160, and the storage 160 may store the created calorific value calculation formula. In this case, the measurement module 151 causes the heater element 61 to generate heat at a plurality of different temperatures, and measures a heat loss coefficient value of mixed gas introduced in the chamber 110. Note that, as described with reference to the above-mentioned formula (9), the heat loss coefficient of gas in contact with the heater element 61 may be measured by using the microchip 120.

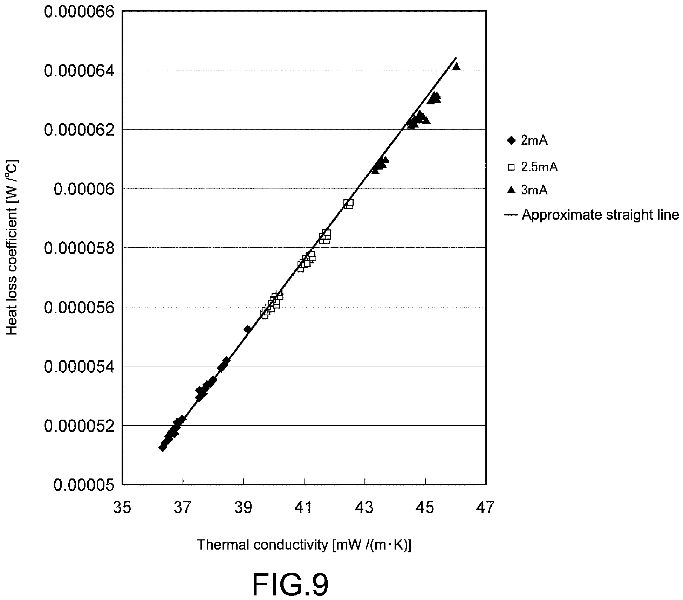

Fig. 9 is a graph showing an example of the relationship between the heat loss coefficient and the thermal conductivity of mixed gas. Note that, in the graph of Fig. 9, the heat loss coefficients and thermal conductivities of mixed gas are plotted where currents of 2 [mA], 2.5 [mA], and 3 [mA] flow in the heater element 61 of Fig. 2 and Fig. 3. As shown in Fig. 9, the heat loss coefficient of mixed gas is in proportion to the thermal conductivity. In view of this, the controller 150 of Fig. 1 may create a calorific value calculation formula where the thermal conductivities of mixed gas at a plurality of different temperatures of the heater element 61 are independent variables, and the calorific value Q is a dependent variable. The storage 160 may store the calorific value calculation formula. In this case, the measurement module 151 causes the heater element 61 to generate heat at a plurality of different temperatures, and measures the thermal conductivity values of mixed gas introduced in the chamber 110.

Further, in the example of this embodiment, the calorific value measurement system 100 of Fig. 1 creates the calorific value calculation formula, and the storage 160 stores the calorific value calculation formula. However, the present invention is not limited to this example. A system other than the calorific value measurement system 100 may create the calorific value calculation formula. The storage 160 of the calorific value measurement system 100 may store the calorific value calculation formula created by the system other than the calorific value measurement system 100.

Next, an example of a behavior of the calorific value measurement system 100 when the calorific value measurement system 100 measures a calorific value Q of mixed gas will be described.

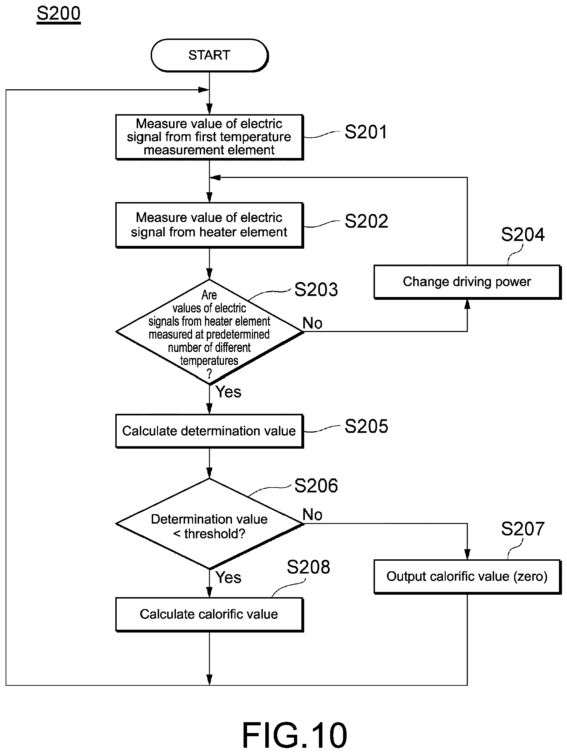

Fig. 10 is a flowchart illustrating an example of a behavior of the calorific value measurement system 100 of Fig. 1 when the calorific value measurement system 100 measures a calorific value Q of mixed gas. For example, in the calorific value measurement system 100 of Fig. 1, when measurement target mixed gas is introduced in the chamber 110 of Fig. 1, the controller 150 reads a program stored in a ROM or the like, and executes calorific value measurement processing S200 of Fig. 10.

First, the measurement module 151 of Fig. 1 measures a value of an electric signal $S_I$ from the first temperature measurement element 62 of Fig. 2 and Fig. 3. The measurement module 151 writes the measured value of the electric signal $S_I$ from the first temperature measurement element 62 in the storage 160. The storage 160 stores the measured value of the electric signal $S_I$ from the first temperature measurement element 62 (S201). In this manner, the value of the electric signal $S_I$ from the first temperature measurement element 62, which is in contact with the gas in the chamber 110, is measured.

Next, the measurement module 151 causes the driving circuit 130 to supply driving power to the heater element 61 of Fig. 2 and Fig. 3. As a result, the heater element 61 generates heat at a predetermined temperature. The measurement module 151 measures the value of the electric signal $S_H$ from the heater element 61, which generates heat at the predetermined temperature. The measurement module 151 writes the measured value of the electric signal $S_H$ from the heater element 61 in the storage 160, whereby the storage 160 stores the measured value (S202). In this manner, the value of the electric signal $S_H$ from the heater element 61, which is in contact with the gas in the chamber 110, is measured.

The measurement module 151 determines if the measurement module 151 has measured the values of the electric signals $S_H$ from the heater element 61 at a predetermined number of different temperatures or not (S203). For example, the measurement module 151 determines if the measurement module 151 has measured values of electric signals $S_H$ at a predetermined number of different temperatures or not based on the number of the measured values of the electric signals $S_H$ from the heater element 61, which is stored in the storage 160.

The predetermined number depends on the number of the independent variables of the electric signals $S_H$ from the heater element 61 of the calorific value calculation formula stored in the storage 160. For example, "5" is preset as the predetermined number.

If it is determined in S203 that the measurement module 151 has not measured the values of the electric signals $S_H$ from the heater element 61 at a predetermined number of different temperatures, the measurement module 151 changes the driving power supplied from the driving circuit 130 (S204), and executes Step S203 again. The measurement module 151 repeats Steps S202 to S204 until the measurement module 151 measures the values of the electric signals $S_H$ from the heater element 61 at a predetermined number of different temperatures. In this manner, the values of the electric signals from the heater element 61, which generates heat at a predetermined number of different temperatures, are measured.

For example, let's say that the predetermined number of different temperatures is "5". First, in Step S202, if the driving circuit 130 supplies the driving power $P_{H1}$ and the heater element 61 generates heat at a temperature $T_{H1}$, the measurement module 151 measures the value of the electric signal $S_{H1}(T_{H1})$ from the heater element 61 when the heater element 61 generates heat at the temperature $T_{H1}$. Next, in Step S204, the driving power $P_{H1}$ supplied from the driving circuit 130 is changed to the driving power $P_{H2}$ larger than the driving power $P_{H1}$. Then, in Step S202, the driving power $P_{H2}$ is supplied to the heater element 61, and the heater element 61 generates heat at the temperature $T_{H2}$. Then the measurement module 151 measures the value of the electric signal $S_{H2}(T_{H2})$ from the heater element 61 when the heater element 61 generates heat at the temperature $T_{H2}$. Next, in Step S204, the driving power $P_{H2}$ supplied from the driving circuit 130 is changed to the driving power $P_{H3}$ larger than the driving power $P_{H2}$. Then, in Step S202, the driving power $P_{H3}$ is supplied to the heater element 61, and the heater element 61 generates heat at the temperature $T_{H3}$. Then the measurement module 151 measures the value of the electric signal $S_{H3}(T_{H3})$ from the heater element 61 when the heater element 61 generates heat at the temperature $T_{H3}$. Next, in Step S204, the driving power $P_{H3}$ supplied from the driving circuit 130 is changed to the driving power $P_{H4}$ larger than the driving power $P_{H3}$. Then, in Step S202, the driving power $P_{H4}$ is supplied to the heater element 61, and the heater element 61 generates heat at the temperature $T_{H4}$. Then the measurement module 151 measures the value of the electric signal $S_{H4}(T_{Ha})$ from the heater element 61 when the heater element 61 generates heat at the temperature $T_{H4}$. Next, in Step S204, the driving power $P_{H4}$ supplied from the driving circuit 130 is changed to the driving power $P_{H5}$ larger than the driving power $P_{H4}$. Then, in Step S202, the driving power $P_{H5}$ is supplied to the heater element 61, and the heater element 61 generates heat at the temperature $T_{H5}$. Then the measurement module 151 measures the value of the electric signal $S_{H5}(T_{H5})$ from the heater element 61 when the heater element 61 generates heat at the temperature $T_{H5}$.

Meanwhile, if it is determined in S203 that the measurement module 151 has measured the values of the electric signals $S_H$ from the heater element 61 at a predetermined number of different temperatures, then it is possible to substitute the predetermined number of measured values of the electric signals $S_H$ from the heater element 61 in a calorific value calculation formula, and to calculate a calorific value of mixed gas.

However, after mixed gas is introduced, gas (e.g., air, etc.) other than the mixed gas may remain in the chamber 110 for a while. Further, mixed gas may leak from the chamber 110, or gas (air) from the outside may flow into the chamber 110 through a clearance or the like of pipes. In those cases, even if a calorific value of gas in the chamber 110 is calculated based on the mixed gas calorific value calculation formula previously created for measurement target mixed gas, it may not be able to correctly calculate a calorific value of gas other than the measurement target mixed gas. In view of this, it is necessary to determine if the gas in the chamber 110 is the measurement target or not before calculating a calorific value based on the calorific value calculation formula.

Here, it is known that the value of the electric signal $S_H$ from the heater element 61 in contact with mixed gas is different from that of the heater element 61 in contact with gas other than the mixed gas because of the difference in the characteristics (e.g., heat loss coefficient, thermal conductivity, etc.) of those kinds of gas. In view of this, it is possible to determine if the gas actually existing in the chamber 110 is the measurement target mixed gas or not by comparing a value based on the measured value of the electric signal $S_H$ from the heater element 61 in contact with the gas in the chamber 110 with a value based on the value of the electric signal $S_H$ from the heater element 61 in contact with the mixed gas.

In view of this, the determination module 152 reads the measured value of the electric signal $S_H$ from the heater element 61, which generates heat at a first temperature, from the storage 160. The determination module 152 calculates a determination value based on the measured value of the electric signal from the heater element 61, which generates heat at the first temperature (S205).

In the above-mentioned example, for example, the first temperature is the temperature $T_{H5}$, i.e., the highest temperature. The determination module 152 calculates a determination value based on the measured value of the electric signal $S_{H5}(T_{H5})$ from the heater element 61 when the heater element 61 generates heat at a temperature $T_{H5}$.

Next, the determination module 152 reads a prestored threshold from the storage 160, compares the determination value calculated in S205 with the threshold, and determines if the determination value is smaller than the threshold (S206).

The threshold is set based on the value of the electric signal $S_H$ from the heater element 61, which generates heat at the first temperature and is in contact with the measurement target mixed gas. Here, both the determination value and the threshold are determined based on the first temperature. The value of the electric signal $S_H$ from the heater element 61, which is in contact with mixed gas and generates heat at the first temperature, may be measured and obtained previously.

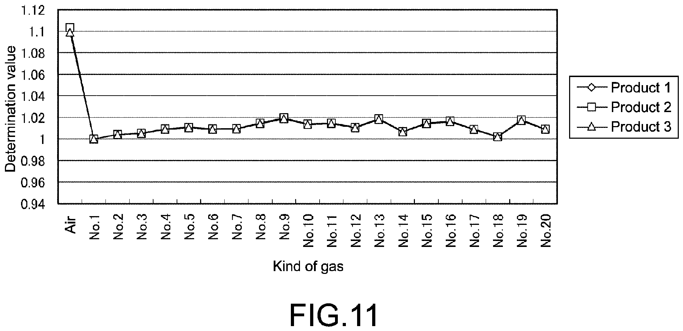

Fig. 11 is a graph showing an example of the relationship between the kinds of gas and determination values. Note that, in consideration of variation between products, each of three calorific value measurement systems 100 (i.e., product 1, product 2, and product 3) measures values of electric signals $S_H$ from the heater element 61, and determination values are calculated. The graph of Fig. 11 shows the calculated determination values. Further, air and twenty kinds of sample mixed gas (No. 1 to No. 20 in Fig. 11) as measurement target mixed gas are prepared. Each of the twenty kinds of sample mixed gas includes, as gas components, at least one of methane ($CH_4$), ethane ($C_2H_6$), propane ($C_3H_8$), butane ($C_4H_{10}$), nitrogen ($N_2$), and carbon dioxide ($CO_2$) of different rates (percentages). For example, the sample mixed gas No. 1 only includes methane. Further, for example, the sample mixed gas No. 10 includes all of methane, ethane, propane, butane, nitrogen ($N_2$), and carbon dioxide. Further, for example, the determination value (vertical axis of Fig. 11) is obtained by dividing the value of an electric signal $S_H$ from the heater element 61, which generates heat at the first temperature and is in contact with gas (horizontal axis), by the value of an electric signal from the heater element 61, which generates heat at the first temperature and is in contact with methane. As shown in Fig. 11, all the determination values of the twenty kinds of sample mixed gas obtained based on the calorific value measurement systems 100 of the product 1, the product 2, and the product 3 are equal to or larger than 1.0 and equal to or smaller than 1.02. Meanwhile, all the determination values of air obtained by using the calorific value measurement systems 100 of the product 1, the product 2, and the product 3 are about 1.1.

In this case, preferably, the threshold for all the calorific value measurement systems 100 of the product 1, the product 2, and the product 3 is set based on values obtained by dividing the values of the electric signals $S_H$ from the heater element 61, which generates heat at the first temperature and is in contact with the twenty kinds of sample mixed gas, by the value of the electric signal $S_H$ from the heater element 61, which generates heat at the first temperature and is in contact with methane. Preferably, the set threshold is larger than the maximum determination value (1.02) when the heater element 61 is in contact with the sample mixed gas, and smaller than the determination value (1.1) when the heater element 61 is in contact with air. For example, the set threshold is about 1.06.

As described above, the determination value based on the measured value of the electric signal $S_H$ from the heater element 61, which is in contact with the gas in the chamber 110 and generates heat at the first temperature, is compared with the threshold. The threshold is set based on the value of the electric signal $S_H$ from the heater element 61, which is in contact with measurement target mixed gas and generates heat at the first temperature (same temperature). As a result, it is possible to distinguish between measurement target mixed gas and gas other than the mixed gas based on the comparison result.

Further, if the determination value is obtained by dividing the value of the electric signal $S_H$ by the value of the electric signal from the heater element 61, which is in contact with one gas component out of a plurality of kinds of gas components in mixed gas, the determination value for each sample mixed gas is about 1, as shown in Fig. 11. Meanwhile, the determination value for air is far different from 1. Because of this, by comparing the determination value with the threshold, it is possible to distinguish between measurement target mixed gas and gas other than the mixed gas easily based on the comparison result. Here, the determination value is obtained by dividing the measured value of the electric signal $S_H$ from the heater element 61, which is in contact with the gas in the chamber 110 and generates heat at the first temperature, by the value of the electric signal $S_H$ from the heater element 61, which is in contact with one gas component out of a plurality of kinds of gas components of the mixed gas. The threshold is set based on the value obtained by dividing the value of the electric signal $S_H$ from the heater element 61, which is in contact with the mixed gas and generates heat at the first temperature, by the electric signal $S_H$ from the heater element 61, which is in contact with the one gas component and generates heat at the first temperature.

Specifically, preferably, the above-mentioned one gas component is a gas component having the highest rate out of a plurality of kinds of gas components of the mixed gas. For example, if natural gas is the measurement target mixed gas, the one gas component is preferably methane, i.e., the main gas component of natural gas. In this case, the determination value is obtained by dividing the measured value of the electric signal $S_H$ from the heater element 61, which is in contact with the gas in the chamber 110 and generates heat at the first temperature, by the value of the electric signal $S_H$ from the heater element 61 in contact with methane. Because of this, the determination value for each mixed gas is about 1. Meanwhile, the determination value for gas other than the mixed gas is far different from 1. Because of this, by comparing the determination value with the threshold, it is possible to distinguish between measurement target mixed gas and gas other than the mixed gas further easily based on the comparison result.

Here, it is known that the difference in the value of the electric signal from the heater element 61 in contact with mixed gas and the value of the electric signal from the heater element 61 in contact with gas other than the mixed gas is larger as the temperature of the heater element 61 is higher. In view of this, the first temperature, which is used to obtain the determination value and the threshold, is preferably the highest temperature that the heater element 61 is capable of reaching. Because of this, by comparing the determination value with the threshold, it is possible to distinguish between measurement target mixed gas and gas other than the mixed gas with a high degree of accuracy based on the comparison result.

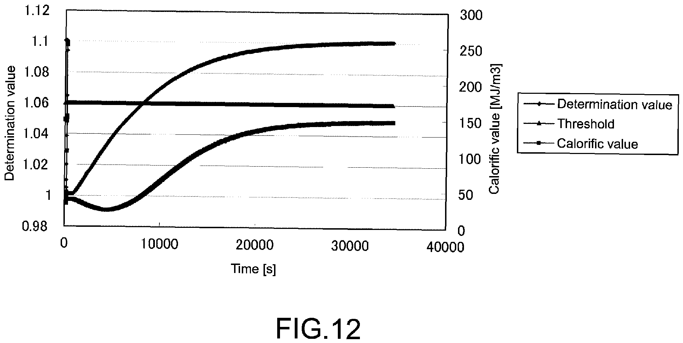

Fig. 12 is a graph showing an example of temporal change of the determination value and a calculated calorific value. In Fig. 12, the horizontal axis shows the elapsed time after the start of introduction of mixed gas to the chamber 110, i.e., the base time (origin). Further, in Fig. 12, similar to the case of Fig. 11, the determination value (left vertical axis) is obtained by dividing the value of an electric signal $S_H$ from the heater element 61, which generates heat at the first temperature and is in contact with gas (horizontal axis), by the value of an electric signal from the heater element 61, which generates heat at the first temperature and is in contact with methane. Further, in Fig. 12, the calorific value (right vertical axis) is a calorific value per unit volume calculated based on the calorific value calculation formula stored in the storage 160. Note that, for comparison, the graph of Fig. 12 shows the threshold (1.06) set in the example of Fig. 11. As shown in Fig. 12, in the beginning of introduction of the mixed gas in the chamber 110, gas (e.g., air, etc.) other than the mixed gas remains in the chamber 110. Because of this, the determination value is about 1.1. At this time, the calorific value Q per unit volume is calculated based on the measured value of the electric signal $S_H$ from the heater element 61 by using the calorific value calculation formula, and the calculated calorific value Q per unit volume is about 148 to 250 [MJ/m$^3$], for example. After that, with the course of time, the air in the chamber 110 is replaced by the mixed gas. At this time, the determination value is about 1.02. The calorific value Q per unit volume is calculated based on the measured value of the electric signal $S_H$ from the heater element 61 by using the calorific value calculation formula, and the calculated calorific value Q per unit volume is an optimum value, i.e., about 37.6 [MJ/m$^3$], for example. Next, for example, after about 850 [s] passes, introduction of the mixed gas in the chamber 110 is stopped. Then, the mixed gas leaks from the chamber 110, and outside gas (air) enters the chamber 110 from a clearance or the like of pipes. As a result, the mixed gas in the chamber 110 is gradually replaced by air. At this time, the calorific value Q per unit volume is calculated based on the measured value of the electric signal $S_H$ from the heater element 61 by using the calorific value calculation formula. The calculated calorific value Q per unit volume temporarily decreases, then increases, and finally reaches about 148 [MJ/m$^3$], for example. During this period, the change rate (inclination) of the determination value is larger than the calorific value Q calculated based on the calorific value calculation formula, and the determination value finally reaches about 1.1. For example, after about 8000 [s] passes, the determination value exceeds the threshold, i.e., 1.06. Meanwhile, after about 8000 [s] passes, the calorific value Q calculated based on the calorific value calculation formula is about 42 to 43 [MJ/m$^3$]. It is difficult to determine if the gas in the chamber 110 is the measurement target mixed gas or not based on the calorific value Q.

Here, with reference to the flowchart of Fig. 10 again, if it is determined in S206 that the determination value is not less than the threshold (i.e., equal to or larger than the threshold), it would appear that not mixed gas but air, for example, is in the chamber 110. In view of this, the determination module 152 does not calculate a calorific value, and outputs the calorific value Q per unit volume, i.e., 0 [MJ/m$^3$] to a display panel or the like of the output apparatus 180 (S207).

Meanwhile, if it is determined in S206 that the determination value is less than the threshold, it would appear that mixed gas is in the chamber 110. In view of this, the calorific value calculation module 153 reads the measured value of the electric signal $S_I$ from the first temperature measurement element 62, the measured values of the electric signals $S_H$ from the heater element 61, which generates heat at a predetermined number of different temperatures, and the mixed gas calorific value calculation formula from the storage 160. The calorific value calculation module 153 substitutes the measured value of the electric signal $S_I$ from the first temperature measurement element 62 and the measured values of the electric signals $S_H$ from the heater element 61, which generates heat at a predetermined number of different temperatures, in the calorific value calculation formula, to thereby calculate the calorific value Q. The calorific value calculation module 153 outputs the calculated calorific value Q per unit volume to a display panel or the like of the output apparatus 180 (S208). As described above, when it is determined that a measurement target gas (i.e., mixed gas) is in the chamber 110, the calorific value calculation module 153 calculates the calorific value of the gas in the chamber 110 based on the measured value of the electric signal $S_H$ from the heater element 61. Because of this, it is possible to prevent from faultily calculating a calorific value based on the mixed gas calorific value calculation formula when it is determined that not mixed gas but gas other than mixed gas is in the chamber 110.

After Steps S207 and S208, the controller 150 returns to Step S201, and repeats Steps S201 to S208 until the calorific value measurement system 100 stops, for example.

According to the example of this embodiment, the determination value is obtained by dividing the measured value of the electric signal $S_H$ from the heater element 61, which generates heat at the first temperature, by the value of the electric signal $S_H$ from the heater element 61, which generates heat at the first temperature and is in contact with one gas component out of a plurality of kinds of gas components of mixed gas. However, a determination value may be obtained by another way as long as the determination value is a ratio between the measured value of the electric signal $S_H$ from the heater element 61, which generates heat at the first temperature, and the value of the electric signal $S_H$ from the heater element 61, which generates heat at the first temperature and is in contact with one gas component out of a plurality of kinds of gas components of mixed gas. For example, a determination value may be obtained by dividing the value of the electric signal $S_H$ from the heater element 61, which generates heat at the first temperature and is in contact with one gas component out of a plurality of kinds of gas components of mixed gas, by the measured value of the electric signal $S_H$ from the heater element 61, which generates heat at the first temperature. In this case, similarly, a threshold is determined based on the value obtained by dividing the value of the electric signal $S_H$ from the heater element 61, which generates heat at the first temperature and is in contact with one gas component out of a plurality of kinds of gas components of mixed gas, by the value of the electric signal $S_H$ from the heater element 61, which generates heat at the first temperature and is in contact with mixed gas. In this manner, a determination value may be a ratio including an inverse ratio, and a threshold may be set based on a ratio including an inverse ratio.

Note that the determination value and the threshold are not limited to the above-mentioned examples. The calorific value measurement system 100 may employ another determination value and another threshold.

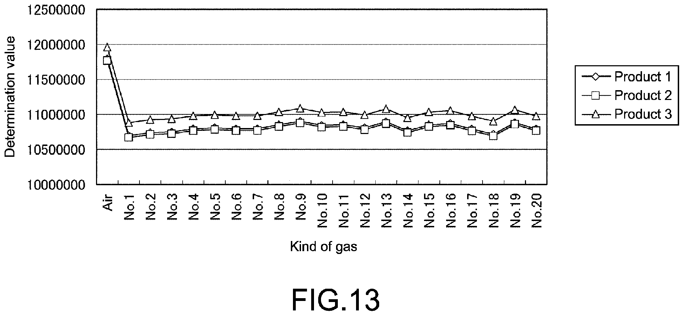

Fig. 13 is a graph showing another example of the relationship between the kinds of gas and a determination value. Note that, similar to the case of Fig. 11, in consideration of variation between products, each of three calorific value measurement systems 100 (i.e., product 1, product 2, and product 3) measures values of electric signals $S_H$ from the heater element 61, and determination values are calculated. The graph of Fig. 13 shows the calculated determination values. Further, air and twenty kinds of sample mixed gas (No. 1 to No. 20 in Fig. 13) as measurement target mixed gas are prepared. Each of the twenty kinds of sample mixed gas includes, as gas components, at least one of methane ($CH_4$), ethane ($C_2H_6$), propane ($C_3H_8$), butane ($C_4H_{10}$), nitrogen ($N_2$), and carbon dioxide ($CO_2$) of different rates (percentages). For example, the sample mixed gas No. 1 only includes methane. Further, for example, the sample mixed gas No. 10 includes all of methane, ethane, propane, butane, nitrogen ($N_2$), and carbon dioxide. Further, the determination value (vertical axis of Fig. 13) is the value of an electric signal $S_H$ from the heater element 61, which generates heat at the first temperature and is in contact with gas (horizontal axis),. As shown in Fig. 13, all the determination values of the twenty kinds of sample mixed gas obtained by using the calorific value measurement systems 100 of the product 1, the product 2, and the product 3 are between about 10700000 and about 11100000. Meanwhile, all the determination values of air obtained by using the calorific value measurement systems 100 of the product 1, the product 2, and the product 3 are between about 11700000 and about 11900000.

In this case, preferably, the threshold for all the calorific value measurement systems 100 of the product 1, the product 2, and the product 3 is set based on values of the electric signals $S_H$ from the heater element 61, which generates heat at the first temperature and is in contact with the twenty kinds of sample mixed gas. Preferably, the set threshold is larger than the maximum determination value (11100000) when the heater element 61 is in contact with the sample mixed gas, and smaller than the determination value (11700000) when the heater element 61 is in contact with air. For example, the set threshold is about 11300000. As described above, the determination value, i.e., the measured value of the electric signal $S_H$ from the heater element 61, which is in contact with the gas in the chamber 110 and generates heat at the first temperature, is compared with the threshold set based on the value of the electric signal $S_H$ from the heater element 61, which is in contact with mixed gas and generates heat at the first temperature. As a result, it is possible to easily distinguish between measurement target mixed gas and gas other than the mixed gas based on the comparison result.

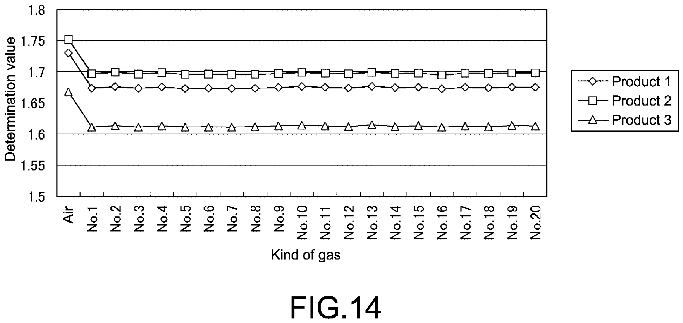

Fig. 14 is a graph showing still another example of the relationship between the kinds of gas and a determination value. Note that, similar to the cases of Fig. 11 and Fig. 12, in consideration of variation between products, each of three calorific value measurement systems 100 (i.e., product 1, product 2, and product 3) measures values of electric signals $S_H$ from the heater element 61, and determination values are calculated. The graph of Fig. 13 shows the calculated determination values. Further, air and twenty kinds of sample mixed gas (No. 1 to No. 20 in Fig. 14) as measurement target mixed gas are prepared. Each of the twenty kinds of sample mixed gas includes, as gas components, at least one of methane ($CH_4$), ethane ($C_2H_6$), propane ($C_3H_8$), butane ($C_4H_{10}$), nitrogen ($N_2$), and carbon dioxide ($CO_2$) of different rates (percentages). For example, the sample mixed gas No. 1 only includes methane. Further, for example, the sample mixed gas No. 10 includes all of methane, ethane, propane, butane, nitrogen ($N_2$), and carbon dioxide. Further, the determination value (vertical axis of Fig. 14) is obtained by dividing the value of an electric signal $S_H$ from the heater element 61, which generates heat at the first temperature and is in contact with gas (horizontal axis), by the value of an electric signal $S_H$ from the heater element 61, which generates heat at a second temperature different from the first temperature and is in contact with gas (horizontal axis). In the above-mentioned example, the second temperature is, for example, the lowest temperature $T_{H1}$. The threshold is obtained by dividing the value of an electric signal $S_H$ from the heater element 61, which generates heat at the first temperature and is in contact with gas (horizontal axis), by the value of the electric signal $S_{H1}(T_{H1})$ from the heater element 61 when the heater element 61 generates heat at the temperature $T_{H1}$. As shown in Fig. 14, all the determination values of the twenty kinds of sample mixed gas obtained by using the calorific value measurement systems 100 of the product 1 is about 1.67, while the determination value of air is about 1.73. Further, all the determination values of the twenty kinds of sample mixed gas obtained by using the calorific value measurement systems 100 of the product 2 is about 1.7, while the determination value of air is about 1.75. Further, all the determination values of the twenty kinds of sample mixed gas obtained by using the calorific value measurement systems 100 of the product 3 is about 1.61, while the determination value of air is about 1.66.

In this case, preferably, the threshold for all the calorific value measurement systems 100 of the product 1, the product 2, and the product 3 is set based on values obtained by dividing the values of the electric signals $S_H$ from the heater element 61, which generates heat at the first temperature and is in contact with the twenty kinds of sample mixed gas, by the values of the electric signals $S_H$ from the heater element 61, which generates heat at the second temperature and is in contact with the twenty kinds of sample mixed gas. Preferably, the set threshold for the calorific value measurement systems 100 of the product 1 is larger than the determination value (1.67) when the heater element 61 is in contact with the sample mixed gas, and smaller than the determination value (1.73) when the heater element 61 is in contact with air. For example, the set threshold is about 1.7. Preferably, the set threshold for the calorific value measurement systems 100 of the product 2 is larger than the determination value (1.7) when the heater element 61 is in contact with the sample mixed gas, and smaller than the determination value (1.75) when the heater element 61 is in contact with air. For example, the set threshold is about 1.72. Preferably, the set threshold for the calorific value measurement systems 100 of the product 3 is larger than the determination value (1.61) when the heater element 61 is in contact with the sample mixed gas, and smaller than the determination value (1.66) when the heater element 61 is in contact with air. For example, the set threshold is about 1.64. As described above, the determination value is obtained by dividing the measured value of the electric signal $S_H$ from the heater element 61, which is in contact with the gas in the chamber 110 and generates heat at the first temperature, by the value of the electric signal $S_H$ of the heater element 61, which is in contact with the gas in the chamber 110 and generates heat at the second temperature different from the first temperature. The threshold is set based on the value obtained by dividing the value of the electric signal $S_H$ from the heater element 61, which generates heat at the first temperature and is in contact with the mixed gas, by the value of the electric signal $S_H$ from the heater element 61, which generates heat at the second temperature different from the first temperature and is in contact with the mixed gas. By comparing the determination value with the threshold, it is possible to distinguish between measurement target mixed gas and gas other than the mixed gas based on the comparison result.

In the example of this embodiment, the determination value is obtained by dividing the measured value of the electric signal $S_H$ from the heater element 61, which generates heat at the first temperature, by the value of the electric signal $S_H$ from the heater element 61, which generates heat at the second temperature different from the first temperature. However, a determination value may be obtained by another way. The determination value may be a ratio between the measured value of the electric signal $S_H$ from the heater element 61, which generates heat at the first temperature, and the value of the electric signal $S_H$ from the heater element 61, which generates heat at the second temperature. For example, a determination value may be obtained by dividing the value of the electric signal $S_H$ from the heater element 61, which generates heat at the second temperature, by the value of the electric signal $S_H$ from the heater element 61, which generates heat at the first temperature. In this case, similarly, a threshold is determined based on the value obtained by dividing the value of the electric signal $S_H$ from the heater element 61, which generates heat at the second temperature and is in contact with the mixed gas, by the value of the electric signal $S_H$ from the heater element 61, which generates heat at the first temperature and is in contact with the mixed gas. In this manner, a determination value may be a ratio including an inverse ratio, and a threshold may be set based on a ratio including an inverse ratio.

## Claims

1.   A calorific value measurement system (100) configured to measure a calorific value of measurement target mixed gas, the system comprising:

   a heater element (61) coming in contact with gas, the heater element being configured to generate heat at a plurality of different temperatures;
   a measurement module (151) configured to measure a value of an electric signal from the heater element;
   a determination module (152) configured
   to compare a determination value with a predetermined threshold, the determination value being based on a measured value of an electric signal from the heater element (61), the heater element (61) generating heat at a first temperature out of the plurality of different temperatures, and
   to determine if the gas is measurement target or not based on the result of the comparison; and
   a calorific value calculation module (153) configured to calculate a calorific value of the gas based on a measured value of an electric signal from the heater element (61) if it is determined that the gas is a measurement target,
   wherein the predetermined threshold is set based on a value of an electric signal from the heater element (61), the heater element generating heat at the first temperature, when the heater element (61) is in contact with a sample mixed gas, and also based on a value of an electric signal from the heater element (61) when the heater element (61) is in contact with the air.

2.   A calorific value measurement system (100) configured to measure a calorific value of measurement target mixed gas, the system comprising:

   a heater element (61) coming in contact with gas, the heater element being configured to generate heat at a plurality of different temperatures;
a measurement module (151) configured to measure a value of an electric signal from the heater element;
a determination module (152) configured

to compare a determination value with a predetermined threshold, the determination value being based on a measured value of an electric signal from the heater element (61), the heater element (61) generating heat at a first temperature out of the plurality of different temperatures, and

to determine if the gas is measurement target or not based on the result of the comparison; and
a calorific value calculation module (153) configured to calculate a calorific value of the gas based on a measured value of an electric signal from the heater element (61) if it is determined that the gas is a measurement target, wherein

the mixed gas includes a plurality of kinds of gas components,
the determination value is a ratio between a measured value of an electric signal from the heater element (61), the heater element (61) generating heat at the first temperature, and a value of an electric signal from the heater element (61), the heater element (61) generating heat at the first temperature and being in contact with one gas component out of the plurality of kinds of gas components, and

the predetermined threshold is set based on a value of a ratio between a value of an electric signal from the heater element (61), the heater element (61) generating heat at the first temperature and being in contact with the mixed gas, and a value of an electric signal from the heater element (61), the heater element (61) generating heat at the first temperature and being in contact with the one gas component, wherein, in particular, the one gas component is a gas component having the highest rate out of the plurality of kinds of gas components,

or wherein
the determination value is a ratio between a measured value of an electric signal from the heater element (61), the heater element (61) generating heat at the first temperature, and a measured value of an electric signal from the heater element (61), the heater element (61) generating heat at a second temperature out of the plurality of different temperatures, and

the predetermined threshold is set based on a value of a ratio between a value of an electric signal from the heater element (61), the heater element (61) generating heat at the first temperature and being in contact with the mixed gas, and a value of an electric signal from the heater element (61), the heater element (61) generating heat at the second temperature and being in contact with the mixed gas.

3. The calorific value measurement system (100) according to claim 1, wherein
the determination value is a measured value of an electric signal from the heater element, the heater element (61) generating heat at the first temperature.

4. The calorific value measurement system (100) according to any one of claims 1 to 3, wherein
the first temperature is the highest temperature out of the plurality of different temperatures.

5. The calorific value measurement system (100) according to any one of claims 1 to 4, wherein
the mixed gas is natural gas.

6. A method of measuring a calorific value, the method measuring a calorific value of measurement target mixed gas, the method comprising:

measuring a value of an electric signal from a heater element (61) coming in contact with gas, the heater element (61) being configured to generate heat at a plurality of different temperatures;
comparing a determination value with a predetermined threshold, the determination value being based on a measured value of an electric signal from the heater element (61), the heater element (61) generating heat at a first temperature out of the plurality of different temperatures, and determining if the gas is measurement target or not based on the result of the comparison; and
calculating a calorific value of the gas based on a measured value of an electric signal from the heater element (61) if it is determined that the gas is a measurement target, wherein
the predetermined threshold is set based on a value of an electric signal from the heater element (61), the heater element (61) generating heat at the first temperature, when the heater element (61) is in contact with a sample mixed gas, and
also based on a value of an electric signal from the heater element (61) when the heater element (61) is in contact with the air.

7. A method of measuring a calorific value, the method measuring a calorific value of measurement target mixed gas, the method comprising:

measuring a value of an electric signal from a heater element (61) coming in contact with gas, the heater element (61) being configured to generate heat at a plurality of different temperatures;

comparing a determination value with a predetermined threshold, the determination value being based on a measured value of an electric signal from the heater element (61), the heater element (61) generating heat at a first temperature out of the plurality of different temperatures, and determining if the gas is measurement target or not based on the result of the comparison; and

calculating a calorific value of the gas based on a measured value of an electric signal from the heater element (61) if it is determined that the gas is a measurement target, wherein

the mixed gas includes a plurality of kinds of gas components,

the determination value is a ratio between a measured value of an electric signal from the heater element (61), the heater element (61) generating heat at the first temperature, and a value of an electric signal from the heater element (61),

the heater element (61) generating heat at the first temperature and being in contact with one gas component out of the plurality of kinds of gas components, and

the predetermined threshold is set based on a value of a ratio between a value of an electric signal from the heater element (61), the heater element (61) generating heat at the first temperature and being in contact with the mixed gas,

and a value of an electric signal from the heater element (61), the heater element (61) generating heat at the first temperature and being in contact with the one gas component, or wherein

the determination value is a ratio between a measured value of an electric signal from the heater element (61), the heater element (61) generating heat at the first temperature, and a measured value of an electric signal from the heater element (61), the heater element (61) generating heat at a second temperature out of the plurality of different temperatures, and

the predetermined threshold is set based on a value of a ratio between a value of an electric signal from the heater element (61), the heater element (61) generating heat at the first temperature and being in contact with the mixed gas,

and a value of an electric signal from the heater element (61), the heater element (61) generating heat at the second temperature and being in contact with the mixed gas.

8. The method of measuring a calorific value according to claim 7, wherein
the one gas component is a gas component having the highest rate out of the plurality of kinds of gas components.

9. The method of measuring a calorific value according to claim 6, wherein
the determination value is a measured value of an electric signal from the heater element, the heater element (61) generating heat at the first temperature.

10. The method of measuring a calorific value according to any one of claims 6 to 9, wherein
the first temperature is the highest temperature out of the plurality of different temperatures.

11. The method of measuring a calorific value according to any one of claims 6 to 10, wherein
the mixed gas is natural gas.

**Patentansprüche**

1. Brennwertmesssystem (100), das konfiguriert ist, um einen Brennwert eines Messziel-Mischgases zu messen, wobei das System Folgendes umfasst:

ein Heizelement (61), das mit Gas in Kontakt kommt, wobei das Heizelement konfiguriert ist, um Wärme mit einer Vielzahl von verschiedenen Temperaturen zu erzeugen;

ein Messmodul (151), das konfiguriert ist, um einen Wert eines elektrischen Signals von dem Heizelement zu messen;

ein Bestimmungsmodul (152), das konfiguriert ist, um einen Bestimmungswert mit einem vorbestimmten Schwellenwert zu vergleichen, wobei der Bestimmungswert auf einem gemessenen Wert eines elektrischen Signals vom Heizelement (61) basiert, wobei das Heizelement (61) Wärme mit einer ersten Temperatur aus der Vielzahl von verschiedenen Temperaturen erzeugt, und um zu bestimmen, ob das Gas ein Messziel ist oder nicht, basierend auf dem Ergebnis des Vergleichs; und

ein Brennwert-Berechnungsmodul (153), das konfiguriert ist, um einen Brennwert des Gases basierend auf einem gemessenen Wert eines elektrischen Signals vom Heizelement (61) zu berechnen, wenn bestimmt wird, dass das Gas ein Messziel ist, wobei der vorbestimmte Schwellenwert basierend auf einem Wert eines elektrischen Signals vom Heizelement (61) eingestellt wird, wobei das Heizelement Wärme mit der ersten Temperatur erzeugt, wenn das Heizelement (61) mit einem Probemischgas in Kontakt ist, und auch basierend auf einem Wert eines elektrischen Signals vom Heizelement (61), wenn das Heizelement (61) mit der Luft in Kontakt ist.

2. Brennwertmesssystem (100), das konfiguriert ist, um einen Brennwert eines Messziel-Mischgases zu messen, wobei das System Folgendes umfasst:

ein Heizelement (61), das mit Gas in Kontakt kommt, wobei das Heizelement konfiguriert ist, um Wärme mit einer Vielzahl von verschiedenen Temperaturen zu erzeugen;
ein Messmodul (151), das konfiguriert ist, um einen Wert eines elektrischen Signals vom Heizelement zu messen;
ein Bestimmungsmodul (152), das konfiguriert ist, um einen Bestimmungswert mit einem vorbestimmten Schwellenwert zu vergleichen, wobei der Bestimmungswert auf einem gemessenen Wert eines elektrischen Signals vom Heizelement (61) basiert, wobei das Heizelement (61) Wärme mit einer ersten Temperatur aus der Vielzahl von verschiedenen Temperaturen erzeugt, und um zu bestimmen, ob das Gas ein Messziel ist oder nicht, basierend auf dem Ergebnis des Vergleichs; und
ein Brennwert-Berechnungsmodul (153), das konfiguriert ist, um einen Brennwert des Gases basierend auf einem gemessenen Wert eines elektrischen Signals vom Heizelement (61) zu berechnen, wenn bestimmt wird, dass das Gas ein Messziel ist, wobei
das Mischgas eine Vielzahl von Arten von Gaskomponenten umfasst,

wobei der Bestimmungswert ein Verhältnis zwischen einem gemessenen elektrischen Signal vom Heizelement (61), wobei das Heizelement (61) Wärme mit der ersten Temperatur erzeugt, und einem Wert eines elektrischen Signals vom Heizelement (61) ist, wobei das Heizelement (61) Wärme mit der ersten Temperatur erzeugt und in Kontakt mit einer Gaskomponente aus der Vielzahl von Arten von Gaskomponenten ist, und der vorbestimmte Schwellenwert basierend auf einem Wert eines Verhältnisses zwischen einem Wert eines elektrischen Signals vom Heizelement (61), wobei das Heizelement (61) Wärme mit der ersten Temperatur erzeugt und in Kontakt mit dem Mischgas ist, und einem Wert eines elektrischen Signals vom Heizelement (61) eingestellt ist, wobei das Heizelement (61) Wärme mit der ersten Temperatur erzeugt und in Kontakt mit der einen Gaskomponente ist, wobei insbesondere die eine Gaskomponente eine Gaskomponente mit der höchsten Rate aus der Vielzahl von Arten von Gaskomponenten ist, oder wobei der Bestimmungswert ein Verhältnis zwischen einem gemessenen Wert eines elektrischen Signals vom Heizelement (61), wobei das Heizelement (61) Wärme mit der ersten Temperatur erzeugt und einem gemessenen Wert eines elektrischen Signals vom Heizelement (61) ist, wobei das Heizelement (61) Wärme mit einer zweiten Temperatur aus der Vielzahl von verschiedenen Temperaturen erzeugt, und wobei der vorbestimmte Schwellenwert basierend auf einem Wert eines Verhältnisses zwischen einem Wert eines elektrischen Signals vom Heizelement (61), wobei das Heizelement (61) Wärme mit der ersten Temperatur erzeugt und in Kontakt mit dem Mischgas ist, und einem Wert eines elektrischen Signals vom Heizelement (61) eingestellt ist, wobei das Heizelement (61) Wärme mit der zweiten Temperatur erzeugt und in Kontakt mit dem Mischgas ist.

3. Brennwert-Messsystem (100) nach Anspruch 1, wobei der Bestimmungswert ein gemessener Wert eines elektrischen Signals vom Heizelement ist, wobei das Heizelement (61) Wärme mit der ersten Temperatur erzeugt.

4. Brennwert-Messsystem (100) nach einem der Ansprüche 1 bis 3, wobei die erste Temperatur die höchste Tempertur aus der Vielzahl von verschiedenen Temperaturen ist.

5. Brennwert-Messsystem (100) nach einem der Ansprüche 1 bis 4, wobei das Mischgas Erdgas ist.

6. Verfahren zur Messung eines Brennwerts, wobei das Verfahren einen Brennwert eines Messziel-Mischgases misst, wobei das System Folgendes umfasst:

Messen eines Werts eines elektrischen Signals von einem Heizelement (61), das mit Gas in Kontakt kommt, wobei das Heizelement (61) konfiguriert ist, um Wärme mit einer Vielzahl von verschiedenen Temperaturen zu erzeugen;

Vergleichen eines Bestimmungswerts mit einem vorbestimmten Schwellenwert, wobei der Bestimmungswert auf einem gemessenen Wert eines elektrischen Signals vom Heizelement (61) basiert, wobei das Heizelement (61) Wärme mit einer ersten Temperatur aus der Vielzahl von verschiedenen Temperaturen erzeugt, und Bestimmen, ob das Gas ein Messziel ist oder nicht, basierend auf dem Ergebnis des Vergleichs; und

Berechnen eines Brennwerts des Gases basierend auf einem gemessenen Wert eines elektrischen Signals vom Heizelement (61), wenn bestimmt wird, dass das Gas ein Messziel ist, wobei der vorbestimmte Schwellenwert basierend auf einem Wert eines elektrischen Signals vom Heizelement (61) eingestellt wird, wobei das Heizelement (61) Wärme mit der ersten Temperatur erzeugt, wenn das Heizelement (61) mit einem Probemischgas in Kontakt ist, und auch basierend auf einem Wert eines elektrischen Signals vom Heizelement (61), wenn das Heizelement (61) mit der Luft in Kontakt ist.

7. Verfahren zur Messung eines Brennwerts, wobei das Verfahren einen Brennwert eines Messziel-Mischgases misst, wobei das Verfahren Folgendes umfasst:

   Messen eines Werts eines elektrischen Signals von einem Heizelement (61), das mit Gas in Kontakt kommt, wobei das Heizelement (61) konfiguriert ist, um Wärme mit einer Vielzahl von verschiedenen Temperaturen zu erzeugen;

   Vergleichen eines Bestimmungswerts mit einem vorbestimmten Schwellenwert, wobei der Bestimmungswert auf einem gemessenen Wert eines elektrischen Signals vom Heizelement (61) basiert, wobei das Heizelement (61) Wärme mit einer ersten Temperatur aus der Vielzahl von verschiedenen Temperaturen erzeugt, und Bestimmen, ob das Gas ein Messziel ist oder nicht, basierend auf dem Ergebnis des Vergleichs; und Berechnen eines Brennwerts des Gases basierend auf einem gemessenen Wert eines elektrischen Signals vom Heizelement (61), wenn bestimmt wird, dass das Gas ein Messziel ist, wobei das Mischgas eine Vielzahl von Arten von Gaskomponenten umfasst, wobei der Bestimmungswert ein Verhältnis zwischen einem gemessenen Wert eines elektrischen Signals des Heizelements (61), wobei das Heizelement (61) Wärme mit der ersten Temperatur erzeugt, und einem Wert eines elektrischen Signals vom Heizelement (61) ist, wobei das Heizelement (61) Wärme mit der ersten Temperatur erzeugt und mit einer Gaskomponente der Vielzahl von Arten von Gaskomponenten in Kontakt ist, und der vorbestimmte Schwellenwert basierend auf einem Wert eines Verhältnisses zwischen einem Wert eines elektrischen Signals vom Heizelement (61), wobei das Heizelement (61) Wärme mit der ersten Temperatur erzeugt und in Kontakt mit dem Mischgas ist, und einem Wert eines elektrischen Signals vom Heizelement (61) eingestellt ist, wobei das Heizelement (61) Wärme mit der ersten Temperatur erzeugt und in Kontakt mit der einen Gaskomponente ist, und wobei der Bestimmungswert ein Verhältnis zwischen einem gemessenen Wert eines elektrischen Signals vom Heizelement (61), wobei das Heizelement (61) Wärme mit der ersten Temperatur erzeugt und einem gemessenen Wert eines elektrischen Signals vom Heizelement (61) ist, wobei das Heizelement (61) Wärme mit einer zweiten Temperatur aus der Vielzahl von verschiedenen Temperaturen erzeugt, und wobei der vorbestimmte Schwellenwert basierend auf einem Wert eines Verhältnisses zwischen einem Wert eines elektrischen Signals vom Heizelement (61), wobei das Heizelement (61) Wärme mit der ersten Temperatur erzeugt und in Kontakt mit dem Mischgas ist, und einem Wert eines elektrischen Signals vom Heizelement (61) eingestellt ist, wobei das Heizelement (61) Wärme mit der zweiten Temperatur erzeugt und in Kontakt mit dem Mischgas ist.

8. Verfahren zur Messung eines Brennwerts nach Anspruch 7, wobei die eine Gaskomponente eine Gaskomponente mit der höchsten Rate aus der Vielzahl von Arten von Gaskomponenten ist.

9. Verfahren zur Messung eines Brennwerts nach Anspruch 6, wobei der Bestimmungswert ein gemessener Wert eines elektrischen Signals vom Heizelements ist, wobei das Heizelement (61) Wärme mit der ersten Temperatur erzeugt.

10. Verfahren zur Messung eines Brennwerts nach einem der Ansprüche 6 bis 9, wobei die erste Temperatur die höchste Tempertur aus der Vielzahl von verschiedenen Temperaturen ist.

11. Verfahren zur Messung eines Brennwerts nach einem der Ansprüche 6 bis 10, wobei das Mischgas Erdgas ist.

**Revendications**

1. Système de mesure de valeur calorifique (100) configuré pour mesurer une valeur calorifique d'un gaz mixte cible de mesure, le système comprenant :

   un élément chauffant (61) venant en contact avec le gaz, l'élément chauffant étant configuré pour générer de la chaleur à une pluralité de températures différentes ;
   un module de mesure (151) configuré pour mesurer une valeur d'un signal électrique provenant de l'élément chauffant ;
   un module de détermination (152) configuré
   pour comparer une valeur de détermination avec un seuil prédéterminé, la valeur de détermination étant basée sur une valeur mesurée d'un signal électrique provenant de l'élément chauffant (61), l'élément chauffant (61) générant de la chaleur à une première température parmi la pluralité de températures différentes, et
   pour déterminer si le gaz est la cible de mesure ou non sur la base du résultat de la comparaison ; et
   un module de calcul de valeur calorifique (153) configuré pour calculer une valeur calorifique du gaz sur la base d'une valeur mesurée d'un signal électrique provenant de l'élément chauffant (61) s'il est déterminé que le gaz est une cible de mesure, dans lequel
   le seuil prédéterminé est établi sur la base d'une valeur d'un signal électrique provenant de l'élément chauffant (61), l'élément chauffant générant de la chaleur à la première température, lorsque l'élément chauffant (61) est en contact avec un gaz mixte échantillon, et également sur la base d'une valeur d'un signal électrique provenant de l'élément chauffant (61) lorsque l'élément chauffant (61) est en contact avec l'air.

2. Système de mesure de valeur calorifique (100) configuré pour mesurer une valeur calorifique d'un gaz mixte cible de mesure, le système comprenant :

   un élément chauffant (61) venant en contact avec le gaz, l'élément chauffant étant configuré pour générer de la chaleur à une pluralité de températures différentes ;
   un module de mesure (151) configuré pour mesurer une valeur d'un signal électrique provenant de l'élément chauffant ;
   un module de détermination (152) configuré
   pour comparer une valeur de détermination à un seuil prédéterminé, la valeur de détermination étant basée sur une valeur mesurée d'un signal électrique provenant de l'élément chauffant (61), l'élément chauffant (61) générant de la chaleur à une première température parmi la pluralité de températures différentes, et
   pour déterminer si le gaz est une cible de mesure ou non sur la base du résultat de la comparaison ; et
   un module de calcul de valeur calorifique (153) configuré pour calculer une valeur calorifique du gaz sur la base d'une valeur mesurée d'un signal électrique provenant de l'élément chauffant (61) s'il est déterminé que le gaz est une cible de mesure, dans lequel
   le gaz mixte comprend une pluralité de types de composants gazeux,
   la valeur de détermination est un rapport entre une valeur mesurée d'un signal électrique provenant de l'élément chauffant (61), l'élément chauffant (61) générant de la chaleur à la première température, et une valeur d'un signal électrique provenant de l'élément chauffant (61), l'élément chauffant (61) générant de la chaleur à la première température et étant en contact avec un composant gazeux parmi la pluralité de types de composants gazeux, et
   le seuil prédéterminé est établi sur la base d'une valeur d'un rapport entre une valeur d'un signal électrique provenant de l'élément chauffant (61), l'élément chauffant (61) générant de la chaleur à la première température et étant en contact avec le gaz mixte, et une valeur d'un signal électrique provenant de l'élément chauffant (61), l'élément chauffant (61) générant de la chaleur à la première température et étant en contact avec ledit composant gazeux, dans lequel, en particulier, ledit composant gazeux est un composant gazeux ayant le taux le plus élevé parmi la pluralité de types de composants gazeux,

   ou dans lequel
   la valeur de détermination est un rapport entre une valeur mesurée d'un signal électrique provenant de l'élément chauffant (61), l'élément chauffant (61) générant de la chaleur à la première température, et une valeur mesurée d'un signal électrique provenant de l'élément chauffant (61), l'élément chauffant (61) générant de la chaleur à une deuxième température parmi la pluralité de températures différentes, et
   le seuil prédéterminé est établi sur la base d'une valeur d'un rapport entre une valeur d'un signal électrique provenant de l'élément chauffant (61), l'élément chauffant (61) générant de la chaleur à la première température et étant en contact avec le gaz mixte, et une valeur d'un signal électrique provenant de l'élément chauffant (61), l'élément chauffant (61) générant de la chaleur à la deuxième température et étant en contact avec le gaz mixte.

3. Système de mesure de valeur calorifique (100) selon la revendication 1, dans lequel
la valeur de détermination est une valeur mesurée d'un signal électrique provenant de l'élément chauffant, l'élément chauffant (61) générant de la chaleur à la première température.

4. Système de mesure de valeur calorifique (100) selon l'une quelconque des revendications 1 à 3, dans lequel
la première température est la température la plus élevée parmi la pluralité de températures différentes.

5. Système de mesure de valeur calorifique (100) selon l'une quelconque des revendications 1 à 4, dans lequel
le gaz mixte est un gaz naturel.

6. Procédé de mesure d'une valeur calorifique, le procédé mesurant une valeur calorifique d'un gaz mixte cible de mesure, le procédé comprenant :

la mesure d'une valeur d'un signal électrique provenant d'un élément chauffant (61) venant en contact avec le gaz, l'élément chauffant (61) étant configuré pour générer de la chaleur à une pluralité de températures différentes ;
la comparaison d'une valeur de détermination avec un seuil prédéterminé, la valeur de détermination étant basée sur une valeur mesurée d'un signal électrique provenant de l'élément chauffant (61), l'élément chauffant (61) générant de la chaleur à une première température parmi la pluralité de températures différentes, et la détermination si le gaz est la cible de mesure ou non sur la base du résultat de la comparaison ; et
le calcul d'une valeur calorifique du gaz sur la base d'une valeur mesurée d'un signal électrique provenant de l'élément chauffant (61) s'il est déterminé que le gaz est une cible de mesure, dans lequel
le seuil prédéterminé est établi sur la base d'une valeur d'un signal électrique provenant de l'élément chauffant (61), l'élément chauffant (61) générant de la chaleur à la première température, lorsque l'élément chauffant (61) est en contact avec un gaz mixte échantillon, et également sur la base d'une valeur d'un signal électrique provenant de l'élément chauffant (61) lorsque l'élément chauffant (61) est en contact avec l'air.

7. Procédé de mesure d'une valeur calorifique, le procédé mesurant une valeur calorifique d'un gaz mixte cible de mesure, le procédé comprenant :

la mesure d'une valeur d'un signal électrique provenant d'un élément chauffant (61) venant en contact avec le gaz, l'élément chauffant (61) étant configuré pour générer de la chaleur à une pluralité de températures différentes ;
la comparaison d'une valeur de détermination à un seuil prédéterminé, la valeur de détermination étant basée sur une valeur mesurée d'un signal électrique provenant de l'élément chauffant (61), l'élément chauffant (61) générant de la chaleur à une première température parmi la pluralité de températures différentes, et la détermination si le gaz est la cible de mesure ou non sur la base du résultat de la comparaison ; et
le calcul d'une valeur calorifique du gaz sur la base d'une valeur mesurée d'un signal électrique provenant de l'élément chauffant (61) s'il est déterminé que le gaz est une cible de mesure, dans lequel
le gaz mixte comprend une pluralité de types de composants gazeux,
la valeur de détermination est un rapport entre une valeur mesurée d'un signal électrique provenant de l'élément chauffant (61), l'élément chauffant (61) générant de la chaleur à la première température, et une valeur d'un signal électrique provenant de l'élément chauffant (61), l'élément chauffant (61) générant de la chaleur à la première température et étant en contact avec un composant gazeux parmi la pluralité de types de composants gazeux, et
le seuil prédéterminé est établi sur la base d'une valeur d'un rapport entre une valeur d'un signal électrique provenant de l'élément chauffant (61), l'élément chauffant (61) générant de la chaleur à la première température et étant en contact avec le gaz mixte, et une valeur d'un signal électrique provenant de l'élément chauffant (61), l'élément chauffant (61) générant de la chaleur à la première température et étant en contact avec ledit composant gazeux,

ou dans lequel
la valeur de détermination est un rapport entre une valeur mesurée d'un signal électrique provenant de l'élément chauffant (61), l'élément chauffant (61) générant de la chaleur à la première température, et une valeur mesurée d'un signal électrique provenant de l'élément chauffant (61), l'élément chauffant (61) générant de la chaleur à une deuxième température parmi la pluralité de températures différentes, et

le seuil prédéterminé est établi sur la base d'une valeur d'un rapport entre une valeur d'un signal électrique provenant de l'élément chauffant (61), l'élément chauffant (61) générant de la chaleur à la première température et étant en contact avec le gaz mixte, et une valeur d'un signal électrique provenant de l'élément chauffant (61), l'élément chauffant (61) générant de la chaleur à la deuxième température et étant en contact avec le gaz mixte.

8. Procédé de mesure d'une valeur calorifique selon la revendication 7, dans lequel
ledit composant gazeux est un composant gazeux ayant le taux le plus élevé parmi la pluralité de types de composants gazeux.

9. Procédé de mesure d'une valeur calorifique selon la revendication 6, dans lequel
la valeur de détermination est une valeur mesurée d'un signal électrique provenant de l'élément chauffant, l'élément chauffant (61) générant de la chaleur à la première température.

10. Procédé de mesure d'une valeur calorifique selon l'une quelconque des revendications 6 à 9, dans lequel
la première température est la température la plus élevée parmi la pluralité de températures différentes.

11. Procédé de mesure d'une valeur calorifique selon l'une quelconque des revendications 6 à 10, dans lequel
le gaz mixte est un gaz naturel.

FIG.1

FIG.2

FIG.3

FIG.4

FIG.5

FIG.6

FIG.7

FIG.8

FIG.9

S200

FIG.10

FIG.11

FIG.12

FIG.13

FIG.14

**REFERENCES CITED IN THE DESCRIPTION**

**Patent documents cited in the description**

- EP 2010038285 A
- EP 2369338 A2
- JP 5141999 A

**Non-patent literature cited in the description**

- **A.J. SMOLA ; B. SCHOLKOPF.** A Tutorial on Support Vector Regression. *NeuroCOLT Technical Report (NC-TR-98-030),* 1998